# EUROPEAN PATENT SPECIFICATION

(11) **EP 3 189 156 B1**
(45) Date of publication and mention of the grant of the patent: **07.10.2020**
(21) Application number: 15750397.0
(22) Date of filing: 10.08.2015
(51) Int. Cl.: C12Q 1/6886

(54) **THYROID CANCER DIAGNOSIS BY DNA METHYLATION ANALYSIS**
SCHILDDRÜSENKREBSDIAGNOSE DURCH ANALYSE DER DNA-METHYLIERUNG
DIAGNOSTIC DU CANCER DE LA THYROÏDE PAR ANALYSE DE LA MÉTHYLATION DE L'ADN

(30) Priority: 08.08.2014 EP 14180318
(43) Date of publication of application: 12.07.2017
(73) Proprietor: AIT Austrian Institute of Technology GmbH, 1210 Wien (AT)
(72) Inventor: VIERLINGER, Klemens, 1090 Vienna (AT)
(74) Representative: Sonn & Partner Patentanwälte
(86) International application number: PCT/EP2015/068397
(87) International publication number: WO 2016/020551

(56) References cited:
- EP-A1- 1 975 252
- EP-A2- 2 518 166
- WO-A2-2010/056374
- WO-A2-2013/086429
- CA-A1- 2 652 975
- SANDRA RODRÍGUEZ-RODERO ET AL: "DNA Methylation Signatures Identify Biologically Distinct Thyroid Cancer Subtypes", JOURNAL OF CLINICAL ENDOCRINOLOGY & METABOLISM, vol. 98, no. 7, 1 July 2013 (2013-07-01), pages 2811-2821, XP055153990, ISSN: 0021-972X, DOI: 10.1210/jc.2012-3566
- ELLIS RYAN J ET AL: "Genome-wide methylation patterns in papillary thyroid cancer are distinct based on histological subtype and tumor genotype.", THE JOURNAL OF CLINICAL ENDOCRINOLOGY AND METABOLISM FEB 2014, vol. 99, no. 2, February 2014 (2014-02), pages E329-E337, XP008173503, ISSN: 1945-7197
- Illumina Inc.: "Illumina HumanMethylation450 BeadChip (HumanMethylation450_15017482_v.1.1)", , 13 May 2011 (2011-05-13), XP055153942, Retrieved from the Internet: URL:https://www.ebi.ac.uk/arrayexpress/fil es/A-GEOD-13534/A-GEOD-13534.adf.txt [retrieved on 2014-11-19]
- X. M. KEUTGEN ET AL: "A Panel of Four miRNAs Accurately Differentiates Malignant from Benign Indeterminate Thyroid Lesions on Fine Needle Aspiration", CLINICAL CANCER RESEARCH, vol. 18, no. 7, 20 February 2012 (2012-02-20), pages 2032-2038, XP055125465, ISSN: 1078-0432, DOI: 10.1158/1078-0432.CCR-11-2487
- CLARISSA ARAUJO CASSOL ET AL: "Molecular pathology of thyroid cancer", DIAGNOSTIC HISTOPATHOLOGY, vol. 17, no. 3, 14 December 2010 (2010-12-14), pages 124-139, XP028169895, ISSN: 1756-2317, DOI: 10.1016/J.MPDHP.2010.12.006 [retrieved on 2010-12-14]

## Description

The present invention relates to the diagnosis of thyroid cancer and thyroid cancer types based on DNA methylation analysis.

### Background of the invention

Thyroid nodules are widely spread and approximately 20% of the people develop a palpable nodule during live and even up to 70% of the adults have nodules detectable by sonography or autopsy. However, incidence is increasing, mainly to improved diagnostic technologies, the mortality rate decreases and only 5-15% of those nodules prove to be malignant. In Austria for instance malignant nodules have a prevalence of 10-20%, with 9879 new diagnosed cases in 2009, whereas women (n=7321) are at higher risk than man (n=2558). The current method of choice for thyroid nodule diagnostic is fine needle aspiration (FNA), followed by cytological assessment. FNA is recognized as minimal invasive method for the evaluation of the nodules, but the method is far away from perfect in terms of specificity and sensitivity. However, FNA contributes to improved diagnostics as it helps to avoid diagnostic surgery in 62-85% of the patients. Nevertheless, it produces a large number of indeterminate or suspicious results. Patients with such an indeterminate diagnosis should be scheduled for a diagnostic surgery, which goes along with either lobectomy or thyroidectomy in 20-30% of the case due to confirmed malignancy. This leads to an overtreatment of a high number of patients. The introduction of additional diagnostics to avoid unnecessary surgeries would also impact on the health care system which can reduce costs for the health care system at a large scale. In the clinical setting the main challenge is the separation of follicular adenomas (FTA) from follicular carcinomas (FTC), which is very challenging by non-operative diagnostics.

In the past it has been clearly shown that molecular techniques like expression profiling or analyzing the DNA methylation profile can add substantial value to the discrimination of different tumor entities. Vierlinger et al. (BMC Med Genomics 2011, 4:30; and WO 2009/026605) executed a meta-analysis on 4 independent expression datasets for the identification of bi-omarkers for PTC. They showed that the expression profile of a single gene (SERPINA1) provides sufficient information to discriminate PTC from all other major histological thyroid entities with very high precision (sensitivity = 1; specificity = 0.90).

WO 2012/068400 focuses on miRNA expression analysis in the diagnosis of thyroid cancer.

WO 2010/086388 and WO 2010/086389 showed that DNA methylation analysis can be used in the diagnosis of various tumor diseases, especially lung cancer. This was done using a preselected marker set of high relevance in cancer settings.

Ryan et al., The Jour. of Clinic. Endocr. & Metab. 99 (2) (2014): E329-E337 relates to methylated CpG islands in case of PTC.

EP 2 518 166 A2 relates to marker sets for differential expression based thyroid cancer detection.

Probes for genetic testing are used on common platforms marketed by Illumina Inc., such as the Illumina HumanMethylation450 BeadChip (2011).

Rodriguez-Romero et al. (J. Clin. Endocrinol. Metab. 2013, 98:2811-2821) measured DNA methylation in thyroid nodules using a previous platform from Illumina which contained probes for 27000 CpG sites. They report 8613 CpG sites as differentially methylated at a p-value < 0.05, but do not report any diagnostically relevant values (accuracies, AUC-values, etc...). Furthermore, they do not report any combination of markers to be diagnostically relevant. Thus this data was of little practical usability in the clinical setting.

Regardless of these advances, there remains a need for powerful diagnostic methods that provide high reliability and resolution, in particular in distinguishing subtypes of thyroid cancer.

### Summary of the invention

The present invention provides a method of distinguishing a thyroid cancer type or risk thereof, comprising the step of determining the DNA methylation status of at least 3 thyroid cancer genes of a sample of a subject, wherein the at least 3 thyroid cancer genes are selected from three or more of the genes of table 1 and/or table 2, and comparing the methylation status of said genes with a control sample, thereby identifying thyroid cancer DNA in the sample, with the proviso that at least one thyroid cancer gene is selected from TREM1, LRP2, NEK11, ABTB2, ACOT7, ADM, ALOX5, ANKRD22, AXIN2, BHLHE40, C10orf107, C1orf21, C20orf85, CAPS, CHKA, CIITA, CIT, CLN5, COBL, COL22A1, CPLX2, DERL3, DNAH17, DNAH9, ELMO1, ELOVL5, ENO2, FAM20A, FMOD, FRMPD2, GALNT9, GJB6, GRIN2C, HK1, HLA-DOA, HOXD9, IFT140, IL17RD, IP6K3, ITM2C, ITPR1, KCNAB1, KCNN4, KRT80, LILRB1, LIPH, LOC100130238, LRRC23, LYSMD2, MACC1, MICALCL, MINA, MPPED2, MTSS1, MYO1G, NRXN2, NT5C2, NTSR1, PAG1, a PCDHA other than PCDHA13, PCNXL2, PCNXL2, PDZK1IP1, PDZRN4, PERI, PIM3, PRDM11, PRR7, PTHLH, PTPRF, RUNX2, SH2B3, SH3GL3, SLC22A9, SORBS2, SPC24, SUPT3H, SYN2, TFAP2B, TIMP4, TMC6, TMC8, TMEM204, TMOD2, TRIM29, UHRF1, WSCD2, ZSCAN18.

Surprisingly, although it prima facie appeared that Rodriguez-Romero et. al. (supra) provided a thorough investigation of DNA methylation in thyroid cancer using DNA methylation analysis of various hypo- and hypermethylated genes, the genetic methylation markers and methylation patterns identified by the present invention differed significantly from the genes and patterns found by Rodriguez-Romero et al.. The invention further improved prior art attempts by including reliable significance values.

The present invention provides an identifier based on DNA methylation distinguishing thyroid tumor types, including the differentiation between benign (FTA, SN) from malignant (FTC, PTC) cases and distinguishing FTCs from FTAs. The unique genetic markers are not only backed-up by distinguishing DNA methylation patterns but also by their relevance towards mRNA expression. The information provided by the invention is useable in the clinics and can boost the current diagnostic procedures by aiding the cytological assessment not only of indeterminate cases, resulting in higher discrimination power of benign and malignant cases, as well as between FTAs and FTCs. The inventive diagnosis allows improved patient treatment and patient care, towards personalized medicine.

Also disclosed are set comprising probes or primers suitable for the inventive methods.

### Detailed description

The present invention provides methylation specific marker genes for use in methylation analysis and expression analysis in the diagnosis in thyroid cancer. These genes are given in tables 1 and 2. The inventive genes are identified in the tables by Gene Symbols (column 4) and by at least one chromosome positions (columns 2 and 3), which identify preferred potentially methylated nucleotide positions of these genes. The genes are further identified by the probe ids (column 1), which identify a CpG site (at the chromosome positions) in these genes, especially in their regulatory elements.

The one or more nucleic acid that is preferably determined according to the invention is given by reference to the chromosomal locus (column *MAPINFO* in tables 1 and 2), which together with the chromosome number (column *CHR*) refers to the hg19 human genome assembly (version "GRCh/hg19" of Feb. 2009 - see http://genome-euro.ucsc.edu) and identifies an exact position in the genome by a single base). Genetic references herein always refer to the hg19 human genome assembly. Probe sequences (According to probe ids) were made available by Illumina and published by Sandoval et.al. (Sandoval et al. Epigenetics 2011; 6:692 - 702). In the tables, probe ids refer to the sequences represented on the array platform. Each one is used to interrogate a specific CpG site. Chromosome and Mapinfo uniquely identify the location of the first nt of each probe. Methylation of genomic regions near transcription start sites, CpG sites (including CgG islands and CpG shores) and in the first exon is usually associated with reduced gene expression. Methylation at other positions, e.g. in regulatory silencer or elements or re-pressors, may lead to increased gene expression. The present invention is based on an analysis of the methylation status in a genetic region of these genes, such as in the promoter region or other regulatory regions, as well as regions in the open reading frame, including exon or intron portions. Regulatory genetic portion, that are potentially methylated, may be in 5' (upstream) or 3' (downstream) direction of the open reading frame (coding region). Novel genes or novel gene combinations (of which a minority of the individual genes might have been known before) are provided which provide an improvement in thyroid cancer or thyroid condition identification.

The present invention also relates to a set, such as in a kit, of primer and/or probes specific to potentially methylated regions of the inventive genes. Primers are preferably provided as primer pairs. The set is suitable for performing the inventive method, which primers and/or probes are specific for targeting a potentially methylated region in a DNA molecule of one or more of the genes selected from table 1 and/or table 2. Such a set can be a set of PCR primers or a microarray comprising the probes.

The following detailed description relates to all aspects of the invention likewise: The inventive method can be performed by any embodiment of set or the primers and/or probes and the inventive set can be used for or be suitable for, i.e. comprising the means for performing, any of the inventive methods. Of course all described embodiments can be combined with each other as is apparent to a skilled practitioner. Further aspects and embodiments are disclosed in the claims, which can be combined with any embodiment in other claims or described in the detailed description. Where claims require a proviso, subject matter of these claims is also disclosed without said proviso, as it may be disregarded in other embodiments.

The inventive genes of tables 1 and 2 are particularly: ABLIM3, ABTB2, ACOT7, ADM, ALOX5, ANKRD22, AXIN2, BHLHE40, C10orf107, C1orf21, C20orf85, CAPS, CDH13, CHKA, CIITA, CIT, CLN5, COBL, COL22A1, CPLX2, CYB561, DERL3, DNAH17, DNAH9, ELMO1, ELOVL5, ENO2, EPHA10, FAM20A, FMOD, FRMD4A, FRMPD2, GAD1, GALNT9, GJB6, GRIN2C, HK1, HLA-DOA, HOXB4, HOXD9, IFT140, IL17RD, IP6K3, IRF5, ITM2C, ITPR1, KCNAB1, KCNN4, KLK10, KRT80, LILRB1, LIPH, LOC100130238, LRP2, LRRC23, LYSMD2, MACC1, MICALCL, MINA, MIOX, MPPED2, MTSS1, MYO1G, NEK11, NRXN2, NT5C2, NTSR1, PAG1, PCDHA, PCNXL2, PCNXL2, PDZK1IP1, PDZRN4, PERI, PIM3, PRDM11, PRR7, PTHLH, PTPRF, RBP1, RUNX2, SH2B3, SH3GL3, SLC22A9, SORBS2, SPC24, STRA6, SUPT3H, SYN2, TBX2, TFAP2B, TIMP4, TMC6, TMC8, TMEM204, TMOD2, TREM1, TRIM29, UHRF1, WSCD2, ZIC1, ZSCAN18.

All genes of tables 1 and 2 are suitable to distinguish non-cancerous from cancerous indications, wherein table 1 is specialized for grouping non-cancerous and cancerous conditions together (e.g. normal samples, Struma nodosa (SN) and FTA as non-cancerous and PTC and FTC as cancerous) and table 2 is specialized to distinguish FTA and FTC. The markers of table 2 are preferably used to distinguish FTC from FTA in a sample from a patient which/who is suspected of having either FTC or FTA, e.g. as indicated in a previous thyroid or thyroid sample inspection.

The sample may be of a patient who has an enlarged thyroid gland, which may be due to non-cancerous nodes (e.g. SN or FTA) or due to a cancerous condition (e.g. FTA or PTC). The inventive method may also be used on a sample with any thyroid size for risk assessment and prognosis.

Preferably the genes are selected from List 1, which is: ABLIM3, ACOT7, ADM, ALOX5, ANKRD22, AXIN2, BHLHE40, C10orf107, C1orf21, CHKA, CIITA, CIT, COBL, CYB561, DNAH9, ELMO1, EPHA10, FAM20A, FMOD, GJB6, HK1, IFT140, TMEM204, IL17RD, IP6K3, IRF5, ITPR1, KCNAB1, KCNN4, KLK10, KRT80, LIPH, LRP2, MACC1, MICALCL, MINA, MIOX, MPPED2, MTSS1, MYO1G, NEK11, PAG1, PCNXL2, PDZK1IP1, PDZRN4, PIM3, PRDM11, PRR7, RUNX2, SORBS2, SPC24, STRA6, SUPT3H, RUNX2, SYN2, TIMP4, TBX2, TMC6, TMC8, TREM1, UHRF1, WSCD2 (genes of table 1);
and List 2, which is: ACOT7, PTPRF, C1orf21, PCNXL2, GAD1, HOXD9, ITM2C, RBP1, ZIC1, KCNAB1, PCDHA, ABLIM3, CPLX2, HLA-DOA, TREM1, TFAP2B, ELOVL5, COBL, COL22A1, FRMD4A, FRMPD2, NT5C2, ABTB2, SLC22A9, NRXN2, TRIM29, LRRC23, ENO2, PTHLH, WSCD2, SH2B3, CIT, GALNT9, LOC100130238, CLN5, TMOD2, LYSMD2, SH3GL3, CDH13, PERI, HOXB4, AXIN2, GRIN2C, DNAH17, CAPS, SPC24, LILRB1, ZSCAN18, C20orf85, NTSR1, DERL3 (genes of table 2). Gene sequences and further information is available for each of these Gene Symbols at a human genome database, such as the hg19 human genome assembly version "GRCh/hg19" of Feb. 2009.

Especially preferred are markers or marker combinations with high AUC values, such as marker genes TREM1, LRP2 or NEK11, each one independently: alone or in combination with any one of the markers of tables 1 and 2. Especially preferred is the 3-marker combination of TREM1, LRP2 and NEK11, alone or in combination with further markers, especially further markers of tables 1 or 2.

In all aspects and embodiments of the inventions PDCHA, which stands for PCDHA complex (protocadherin alpha and subfamily C), is preferably determined at any one or more (e.g. 1, 2, 3, 4, 5, 6, 7, 8, 9, 10, 11, 12, 13, 14 or 15) of its members selected from PCDHA9, PCDHA6, PCDHA4, PCDHA13, PCDHAC1, PCDHA10, PCDHA8, PCDHA3, PCDHA1, PCDHA5, PCDHA12, PCDHAC2, PCDHA2, PCDHA7 and/or PCDHA11. The PCDHA is preferably a PCDHA other than PCDHA13, or a combination of such other PCDHAs.

Especially preferred, the genes include genes selected from are ACOT7, C1orf21, PCNXL2, KCNAB1, ABLIM3, TREM1, COBL, WSCD2, CIT, AXIN2, SPC24 (genes of both tables 1 and 2).

In further preferred embodiments, the markers used in any embodiment of the invention do not require (or even - but not necessarily - exclude) markers ABLIM3, CYB561, EPHA10, IRF5, KLK10, MIOX, STRA6 and TBX2 (List 3a), or markers ZIC1, PCDHA13, ABLIM3, FRMD4A and HOXB4 (List 3b). In further - combinable with the above - preferred embodiments, also markers GAD1, RBP1, and CDH13 (List 3c) are not prescribed for use or even excluded. In further - combinable with the above - preferred embodiments, also markers KCNAB1 and LRP2 (List 3d) are not prescribed for use or even excluded. Preferably, at least one of genes ABTB2, ACOT7, ADM, ALOX5, ANKRD22, AXIN2, BHLHE40, C10orf107, C1orf21, C20orf85, CAPS, CHKA, CIITA, CIT, CLN5, COBL, COL22A1, CPLX2, DERL3, DNAH17, DNAH9, ELMO1, ELOVL5, ENO2, FAM20A, FMOD, FRMPD2, GALNT9, GJB6, GRIN2C, HK1, HLA-DOA, HOXD9, IFT140, IL17RD, IP6K3, ITM2C, ITPR1, KCNAB1, KCNN4, KRT80, LILRB1, LIPH, LOC100130238, LRP2, LRRC23, LYSMD2, MACC1, MICALCL, MINA, MPPED2, MTSS1, MYO1G, NEK11, NRXN2, NT5C2, NTSR1, PAG1, PCDHA (not PCDHA13), PCNXL2, PCNXL2, PDZK1IP1, PDZRN4, PERI, PIM3, PRDM11, PRR7, PTHLH, PTPRF, RUNX2, SH2B3, SH3GL3, SLC22A9, SORBS2, SPC24, SUPT3H, SYN2, TFAP2B, TIMP4, TMC6, TMC8, TMEM204, TMOD2, TREM1, TRIM29, UHRF1, WSCD2, ZSCAN18 is used or provided for with methylation specific probes or primers in the inventive set (- but not necessarily in any embodiment of the invention; claim 1 is also specifically disclosed without the proviso).

Thus in preferred embodiments the inventive markers are of List 1a: ACOT7, ADM, ALOX5, ANKRD22, AXIN2, BHLHE40, C10orf107, C1orf21, CHKA, CIITA, CIT, COBL, DNAH9, ELMO1, FAM20A, FMOD, GJB6, HK1, IFT140, TMEM204, IL17RD, IP6K3, ITPR1, KCNAB1, KCNN4, KRT80, LIPH, LRP2, MACC1, MICALCL, MINA, MPPED2, MTSS1, MYO1G, NEK11, PAG1, PCNXL2, PDZK1IP1, PDZRN4, PIM3, PRDM11, PRR7, RUNX2, SORBS2, SPC24, SUPT3H, RUNX2, SYN2, TIMP4, TMC6, TMC8, TREM1, UHRF1, WSCD2; and
List 2a: ACOT7, PTPRF, C1orf21, PCNXL2, GAD1, HOXD9, ITM2C, RBP1, KCNAB1, PCDHA (excluding PCDHA13 or all PCDHA members), CPLX2, HLA-DOA, TREM1, TFAP2B, ELOVL5, COBL, COL22A1, FRMPD2, NT5C2, ABTB2, SLC22A9, NRXN2, TRIM29, LRRC23, ENO2, PTHLH, WSCD2, SH2B3, CIT, GALNT9, LOC100130238CLN5, TMOD2, LYSMD2, SH3GL3, CDH13, PERI, AXIN2, GRIN2C, DNAH17, CAPS, SPC24, LILRB1, ZSCAN18, C20orf85, NTSR1, DERL3. List 1a and List and 2a are based on List 1 and List 2, respectively, not including the above mentioned less-preferred markers.

Hyper- or hypomethylation of genes ABLIM3, CYB561, EPHA10, IRF5, KLK10, MIOX, STRA6 and TBX2, or markers ZIC1, PCDHA13, ABLIM3, FRMD4A and HOXB4 in connection with thyroid cancer has been mentioned in Rodriguez-Romero et al. (supra). Regrettably, Rodriguez-Romero et al. did not provide any particular information, like AUC or fold changes or significance that would allow a diagnosis or thyroid cancer state investigation using these markers. The present invention can improve on Rodriguez-Romero et al. by providing improved embodiments with these markers - in other embodiments these markers are not necessarily used. Thus, if these markers are used or included in the set, it is preferred to do this in connection with any one of the preferred inventive embodiments, e.g. as defined in the dependent claims. Such preferred embodiments are e.g. using these markers in combination with any other combination of marker genes of tables 1 and 2) not of List 3a,b,c, possibly further not of List 3d; using these markers in when using probes specific for the potentially methylated regions as defined by the position given in tables 1 and 2; detecting the methylation status of these genes in more than one potentially methylated region, such as 2 or 3 potentially methylated regions, such potentially methylated regions being preferably defined by the positions given in tables 1 and 2; using these markers of list 3a,b,c for distinguishing special thyroid conditions such as FTA from FTC; combining a methylation status analysis with a gene expression analysis; etc..

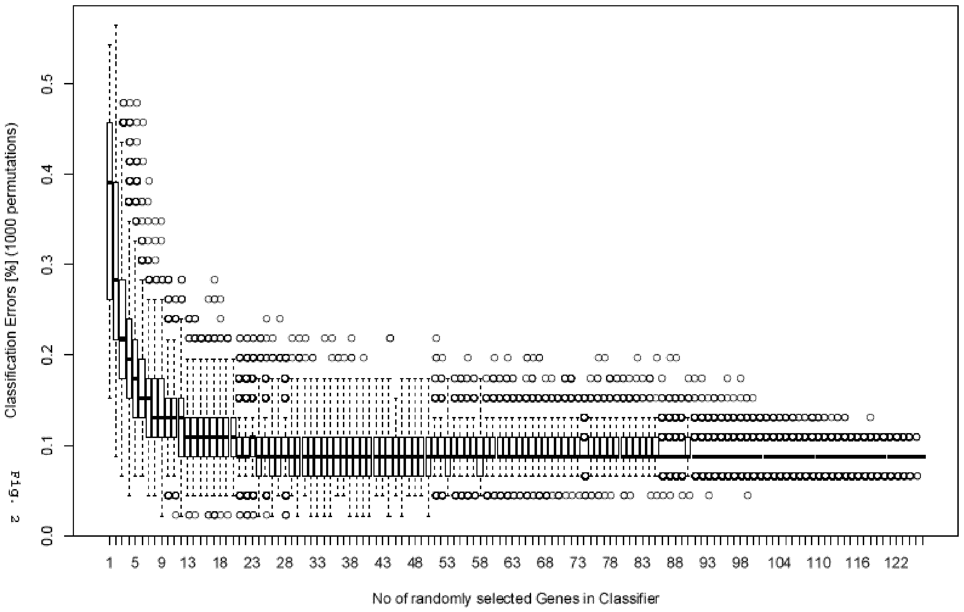

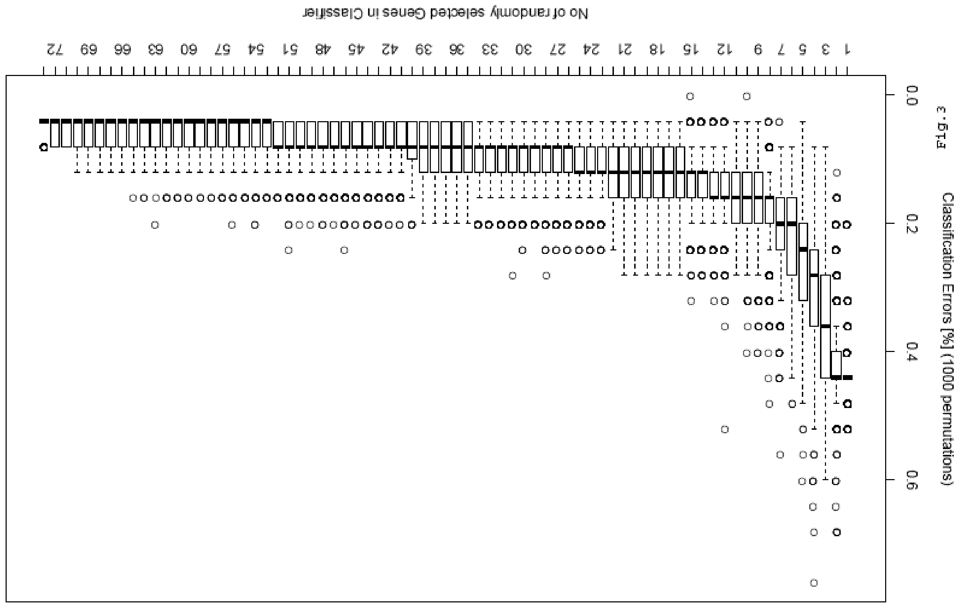

It is particularly preferred to determine more than one gene of the inventive table(s) in any embodiment of the invention, including the set, which may comprises primers and/or probes specific for potentially methylated regions of said more than one genes. Determining the methylation status may comprise determining the methylation status of at least 2, preferably of 2, 3, 4, 5, 6, 7, 8, 9, 10, 11, 12, 13, 14, 15, 16, 17, 18, 19, 20, at least 25, 30, 33, 35, 40, 45, 50 or more of the genes of said table(s) or list(s), e.g. of the combines tables 1 and 2, of table 1, of table 2 or list 1a or list 2a, e.g. of ABLIM3, ABTB2, ACOT7, ADM, ALOX5, ANKRD22, AXIN2, BHLHE40, C10orf107, C1orf21, C20orf85, CAPS, CDH13, CHKA, CIITA, CIT, CLN5, COBL, COL22A1, CPLX2, CYB561, DERL3, DNAH17, DNAH9, ELMO1, ELOVL5, ENO2, EPHA10, FAM20A, FMOD, FRMD4A, FRMPD2, GAD1, GALNT9, GJB6, GRIN2C, HK1, HLA-DOA, HOXB4, HOXD9, IFT140, IL17RD, IP6K3, IRF5, ITM2C, ITPR1, KCNAB1, KCNN4, KLK10, KRT80, LILRB1, LIPH, LOC100130238, LRP2, LRRC23, LYSMD2, MACC1, MICALCL, MINA, MIOX, MPPED2, MTSS1, MYO1G, NEK11, NRXN2, NT5C2, NTSR1, PAG1, PCDHA, PCNXL2, PCNXL2, PDZK1IP1, PDZRN4, PERI, PIM3, PRDM11, PRR7, PTHLH, PTPRF, RBP1, RUNX2, SH2B3, SH3GL3, SLC22A9, SORBS2, SPC24, STRA6, SUPT3H, SYN2, TBX2, TFAP2B, TIMP4, TMC6, TMC8, TMEM204, TMOD2, TREM1, TRIM29, UHRF1, WSCD2, ZIC1, ZSCAN18. It is possible to pick any small number from these subsets or combined set since a distinction between benign and malignant states or the diagnosis of cancer can also be performed with acceptable certainty. For example in a preferred embodiment the inventive set or method comprises at least 3 (or any of the above mentioned numbers) of genes of methylation markers. In fact, these markers can be chosen at random since the inventive tables have been thoroughly compiled to allow just that. Fig. 2 show diagnostic classification probabilities for random selections of any number of markers (x-axis) to distinguish benign vs. malignant states using the markers of table 1. E.g. a set specific for 3 markers has only an error margin of 20%, i.e. 80% of all cases would be classified correctly. An error value of 12% (88% certainty) is achieved with at least 8 members. Fig. 3 show diagnostic classification probabilities for random selections of any number of markers (x-axis) to distinguish FTA vs. FTC states using the markers of table 2. E.g. a set specific for 3 markers has only an error margin of 36%, i.e. 64% of all cases would be classified correctly. An error value of 18% (82% certainty) is achieved with at least 8 members. Both are significant results when taking the generally high uncertainty into consideration that exists in cancer diagnosis (cf. 40% error rate in the standard PSA test in prostate cancer diagnosis).

As said, these numbers are achieved by a random selection of the inventive tables. The result can be even increased by selecting marker combinations with high complementarity to lower the classification error (see. Figs 2 and 3, bottom circles and dashed lines). Such increased complementary markers and genes can be selected by statistical selection algorithms using methylation data from confirmed benign or cancerous states that are to be distinguished.

Such methods include class comparisons wherein a specific p-value is selected, e.g. a p-value below 0.1, preferably below 0.08, more preferred below 0.06, in particular preferred below 0.05, below 0.04, below 0.02, most preferred below 0.01.

Preferably the correlated results for each marker or gene are rated by their correct correlation to thyroid cancer positive state, preferably by p-value test or t-value test or F-test. Rated (best first, i.e. low p- or t-value) markers are the subsequently selected and added to the marker combination until a certain diagnostic value is reached, e.g. the herein mentioned at least 60%, at least 70%, at least 80%, at least 90% or at least 95% (or more) correct classification of thyroid cancer.

Class Comparison procedures include identification of genes that were differentially methylated among the two or more classes using a random-variance t-test. The random-variance t-test is an improvement over the standard separate t-test as it permits sharing information among genes about within-class variation without assuming that all genes have the same variance (Wright G.W. and Simon R, Bioinformatics 19:2448-2455,2003). Genes were considered statistically significant if their p value was less than a certain value, e.g. 0.1 or 0.01. A stringent significance threshold can be used to limit the number of false positive findings. A global test can also be performed to determine whether the methylation profiles differed between the classes by permuting the labels of which arrays corresponded to which classes. For each permutation, the p-values can be re-computed and the number of genes significant at the e.g. 0.01 level can be noted. The proportion of the permutations that give at least as many significant genes as with the actual data is then the significance level of the global test. If there are more than 2 classes, then the "F-test" instead of the "t-test" should be used.

Class Prediction includes the step of specifying a significance level to be used for determining the genes that will be included in the subset. Genes that are differentially methylated between the classes at a univariate parametric significance level less than the specified threshold are included in the set. It doesn't matter whether the specified significance level is small enough to exclude enough false discoveries. In some problems better prediction can be achieved by being more liberal about the gene sets used as features. The sets may be more biologically interpretable and clinically applicable, however, if fewer genes are included.

To prevent increase of the number of the members of the subset, only marker genes with at least a significance value of at most 0.1, preferably at most 0.8, even more preferred at most 0.6, at most 0.5, at most 0.4, at most 0.2, or more preferred at most 0.01 are selected.

Since the combination should be small, it is preferred that not more than 10000, not more than 5000, not more than 2500, not more than 2000, not more than 1500, not more than 1000, not more than 800, not more than 600, or not more than 400, preferably not more than 350, not more than 300, not more than 250, not more than 200, not more than 150, not more than 100, not more than 80, not more than 60, or not more than 40, preferably not more than 30, in particular preferred not more than 20, marker genes are used according to the inventive method or in the inventive set, not counting controls for methylation testing or for gene expression testing. In particular the set of the present invention provides less primer pairs /and or probes than these numbers in order to reduce manufacturing costs in addition to the above reasons.

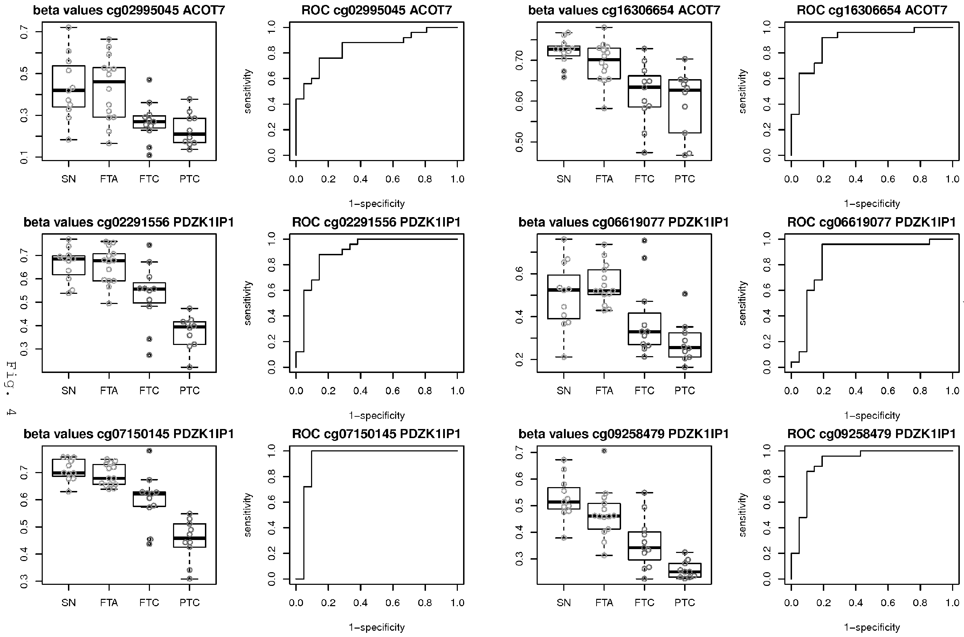

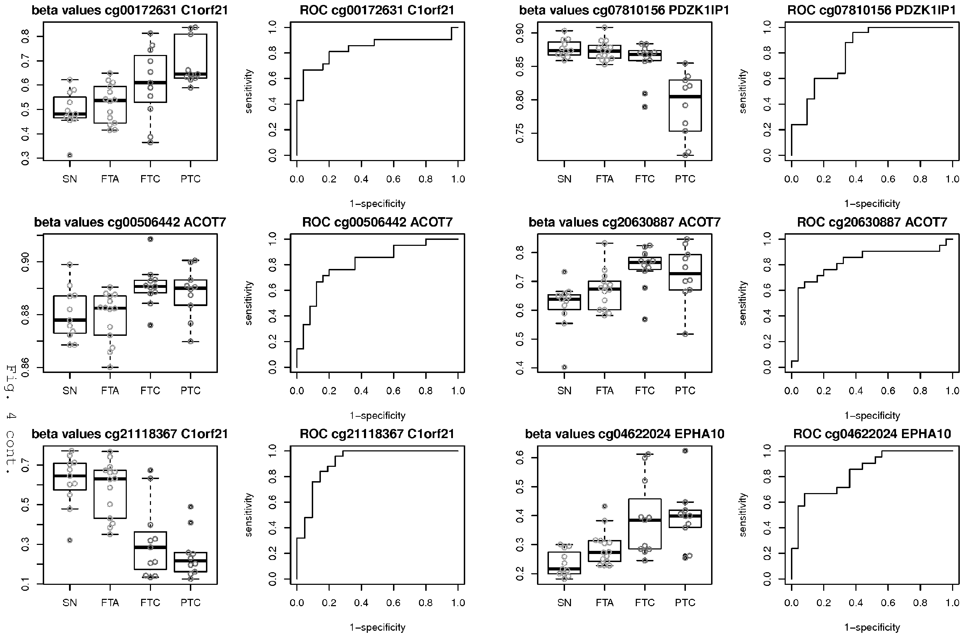

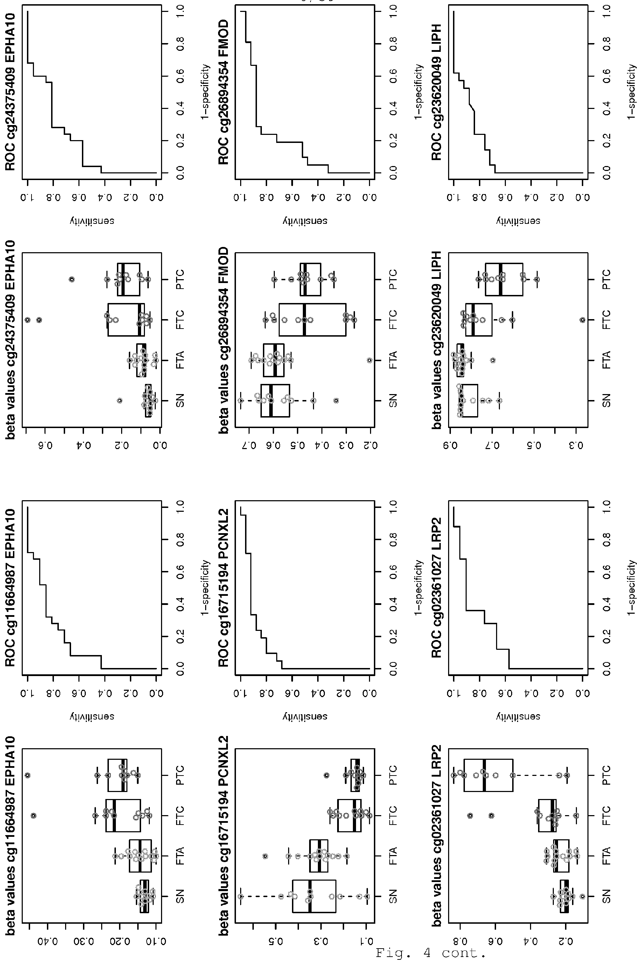

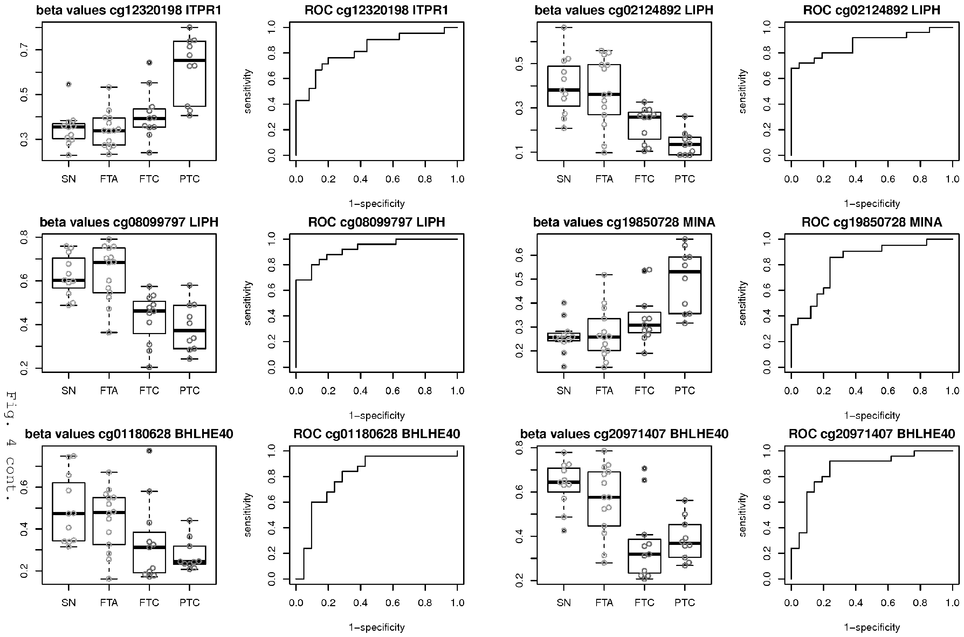

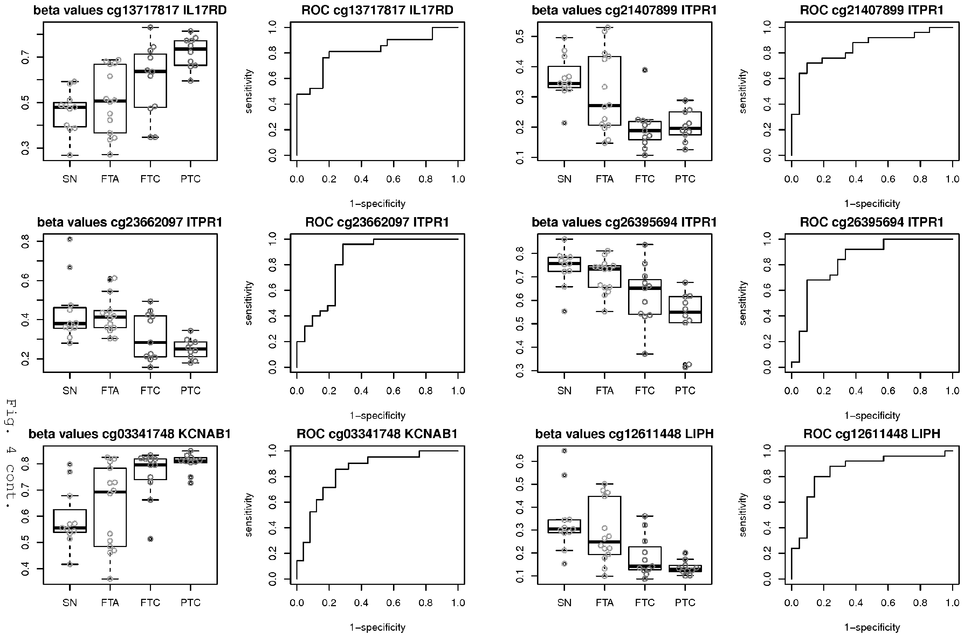

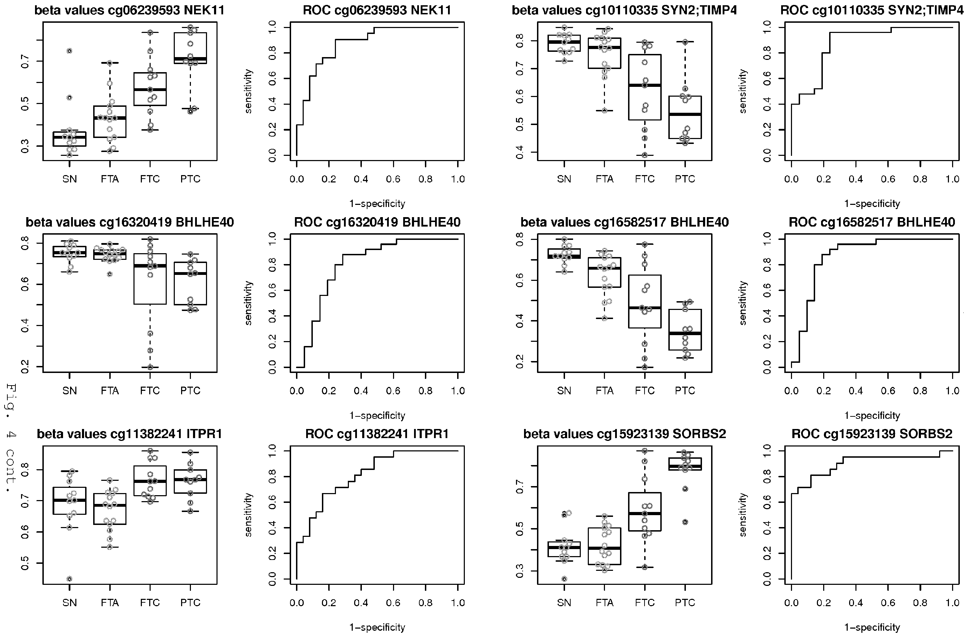

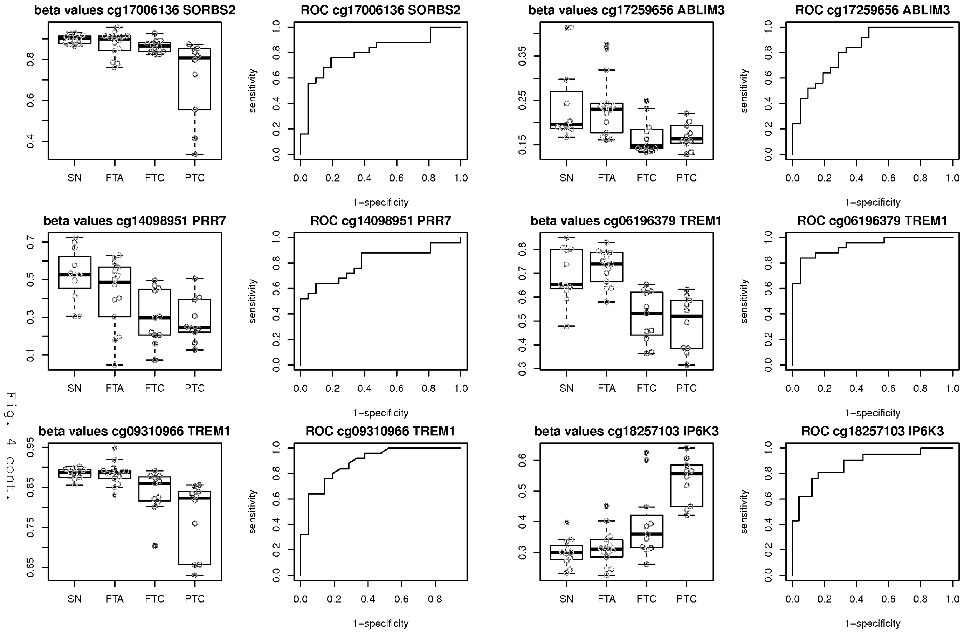

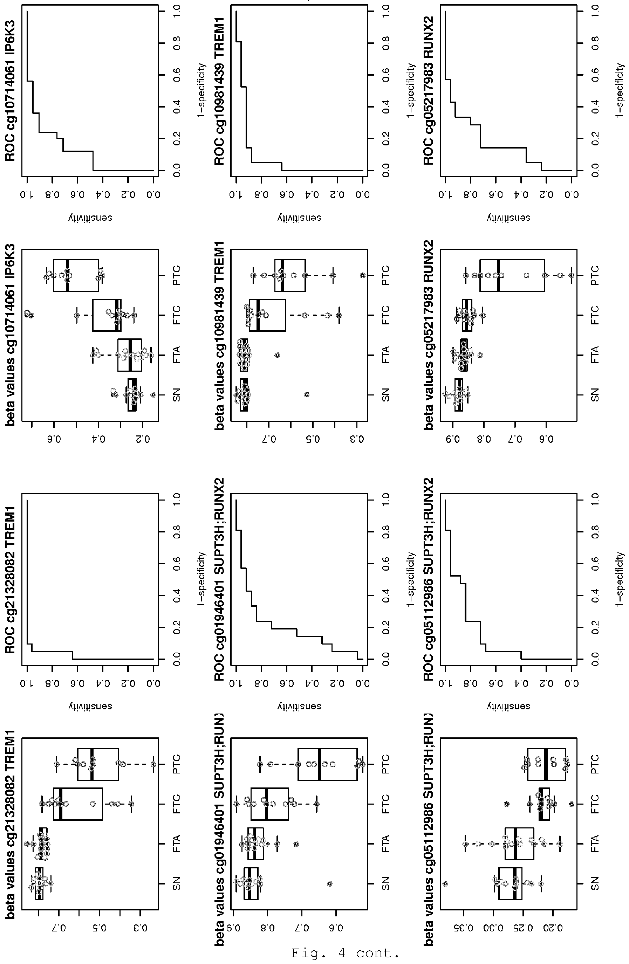

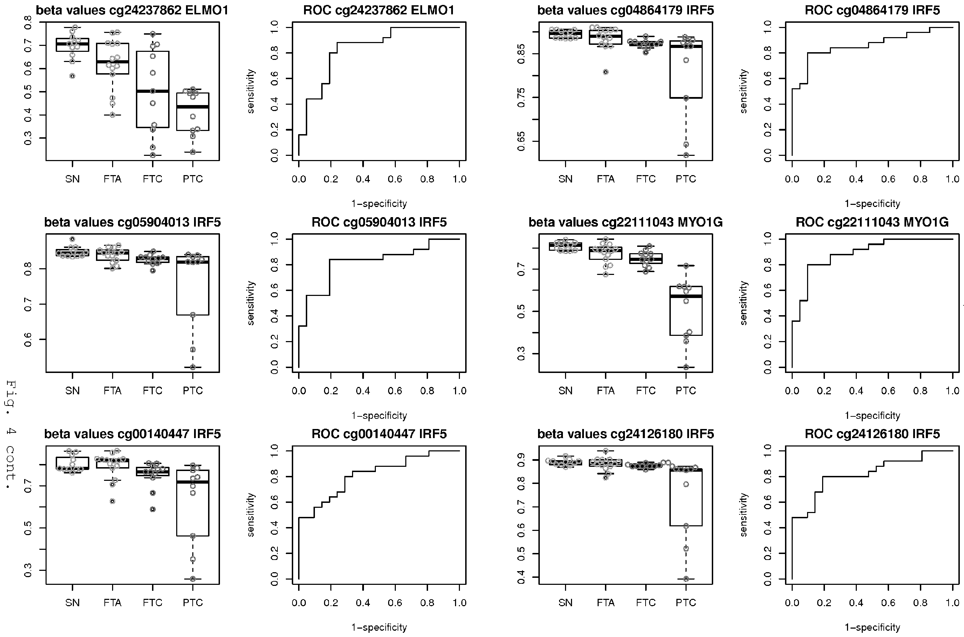

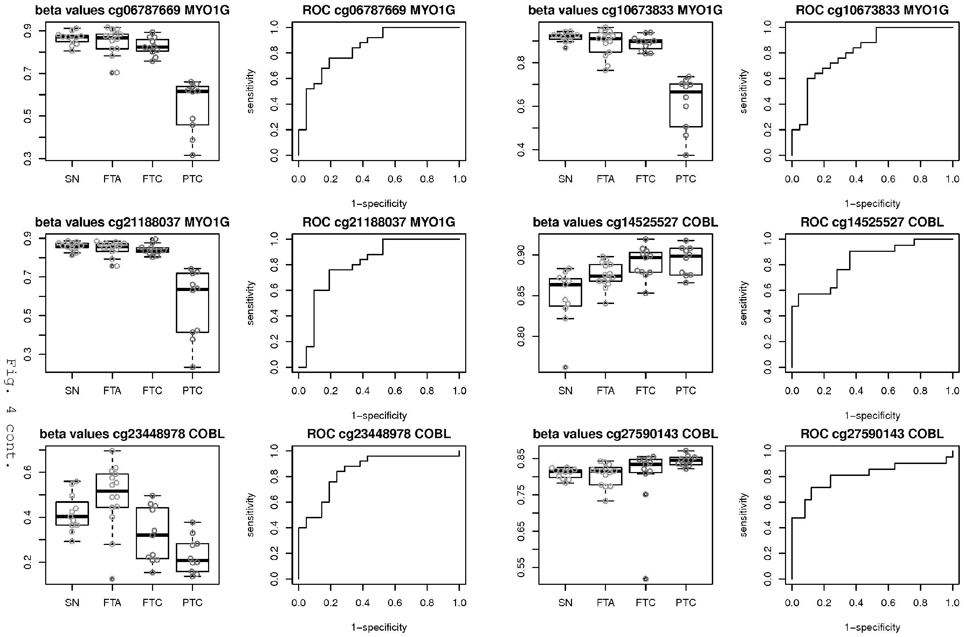

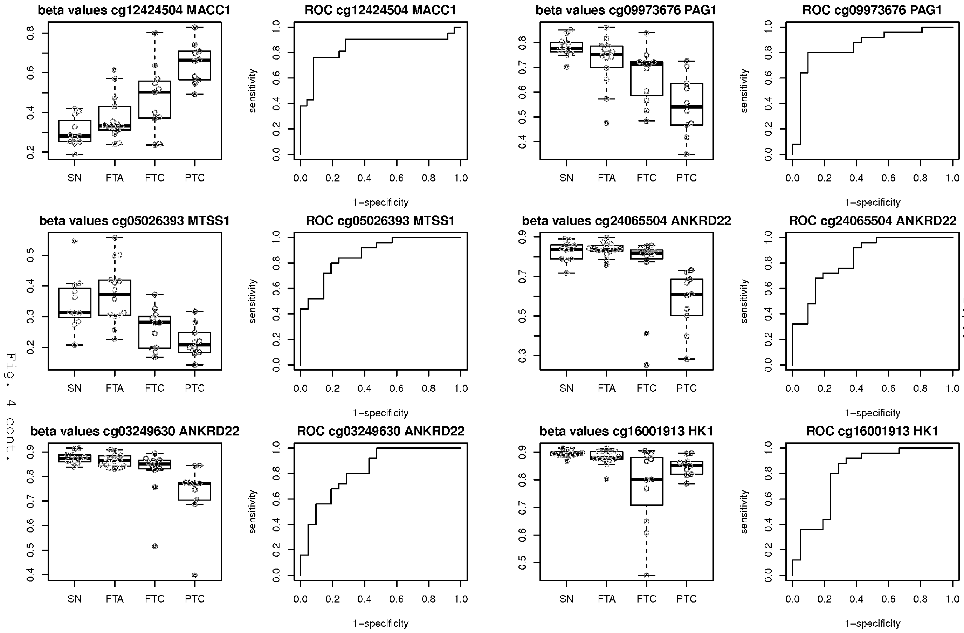

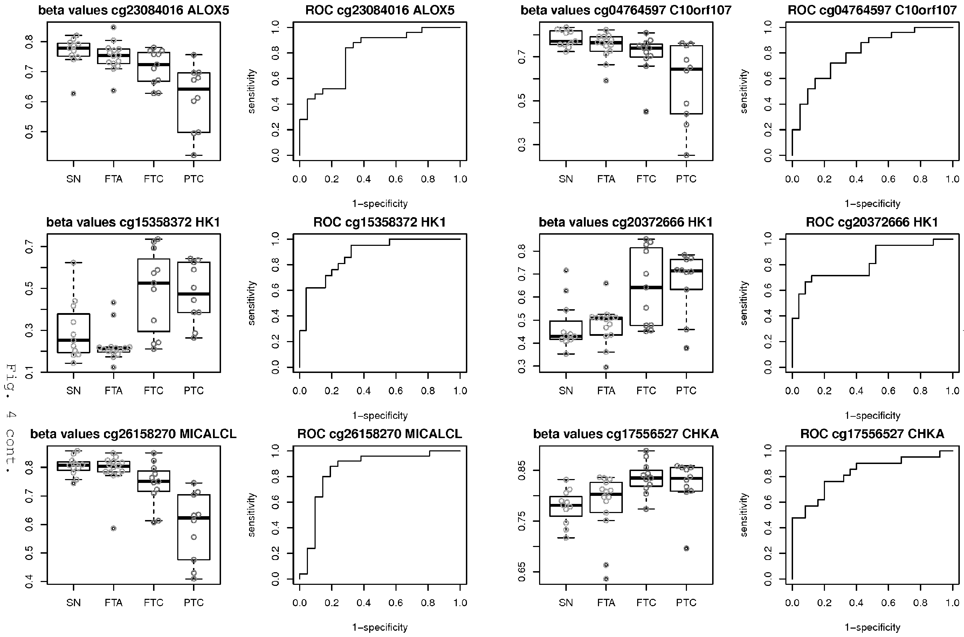

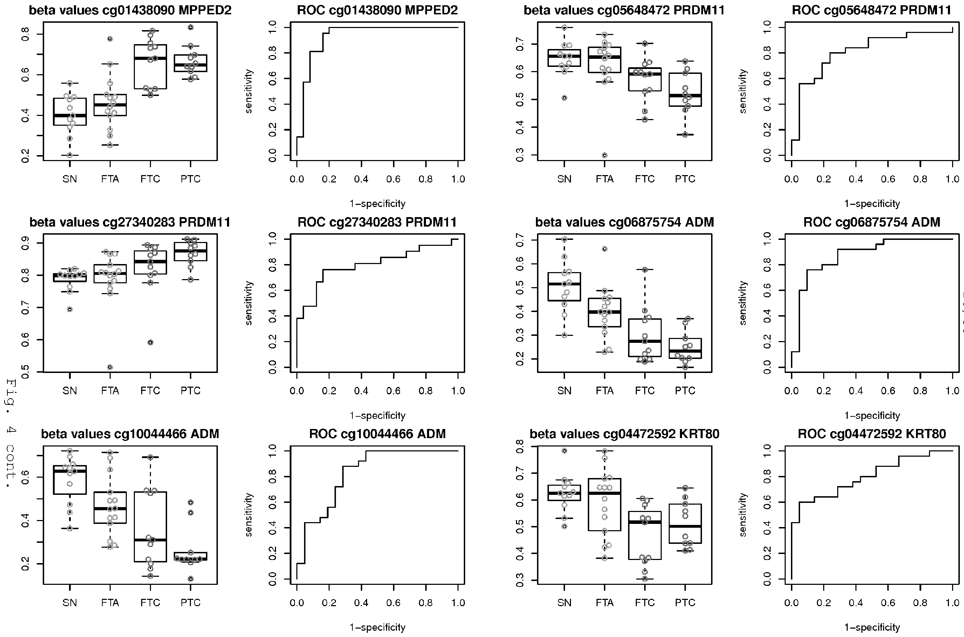

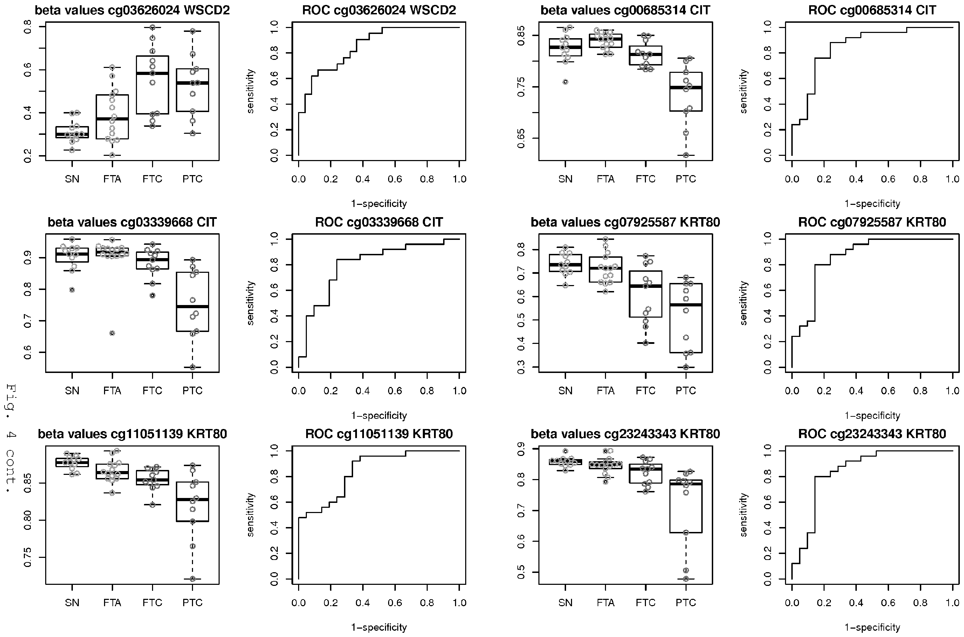

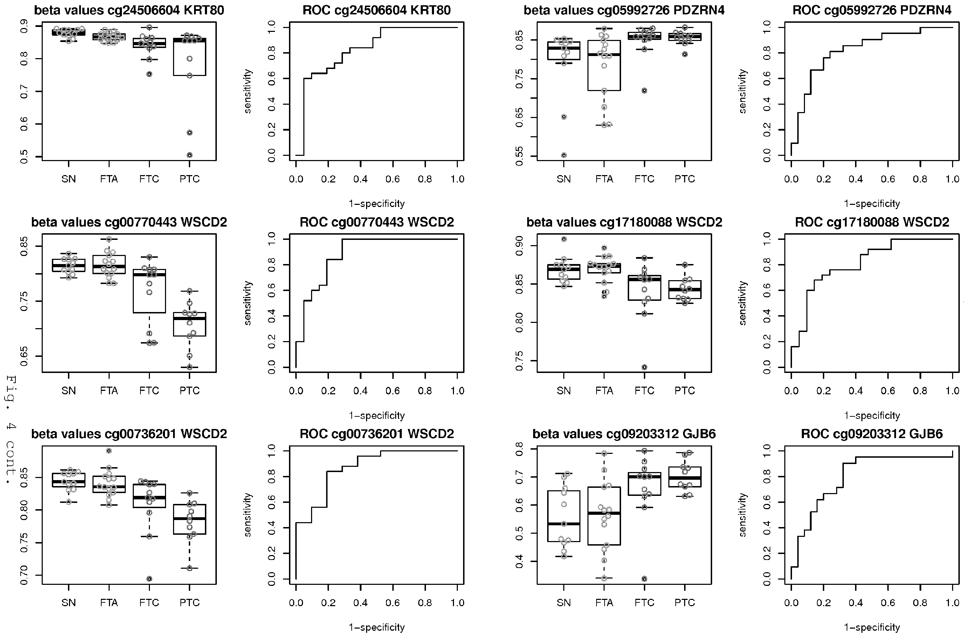

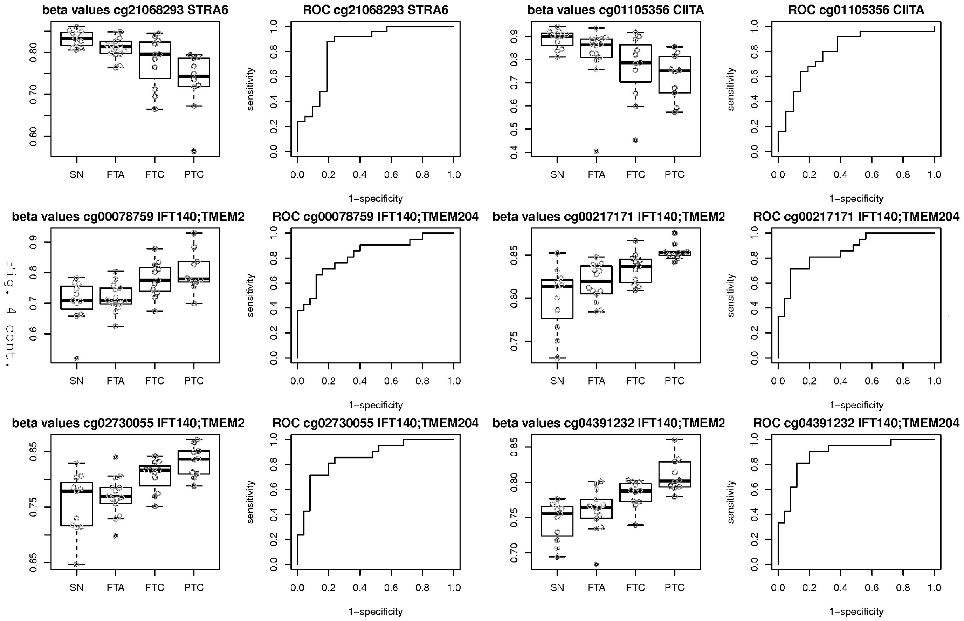

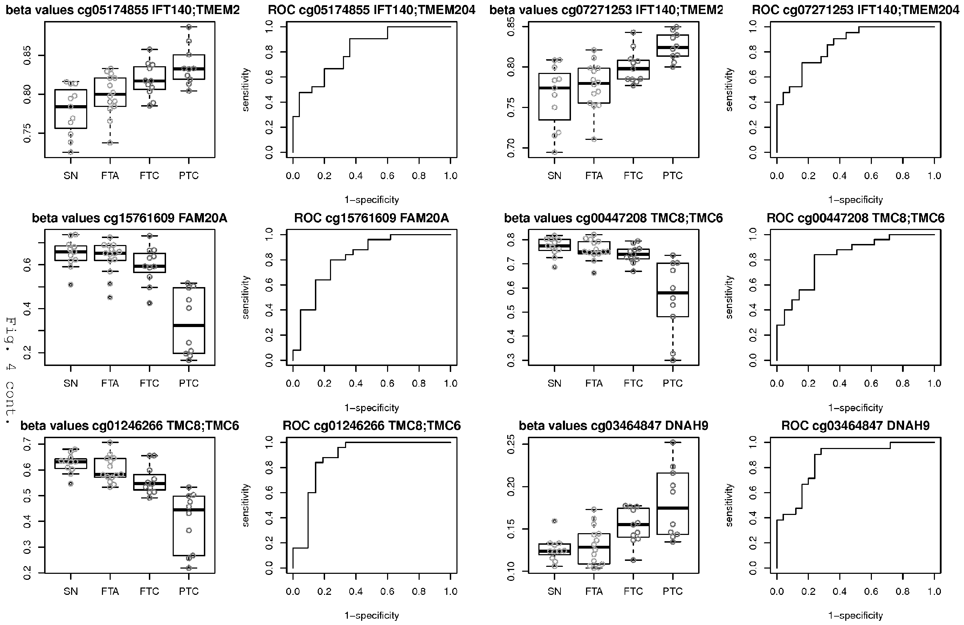

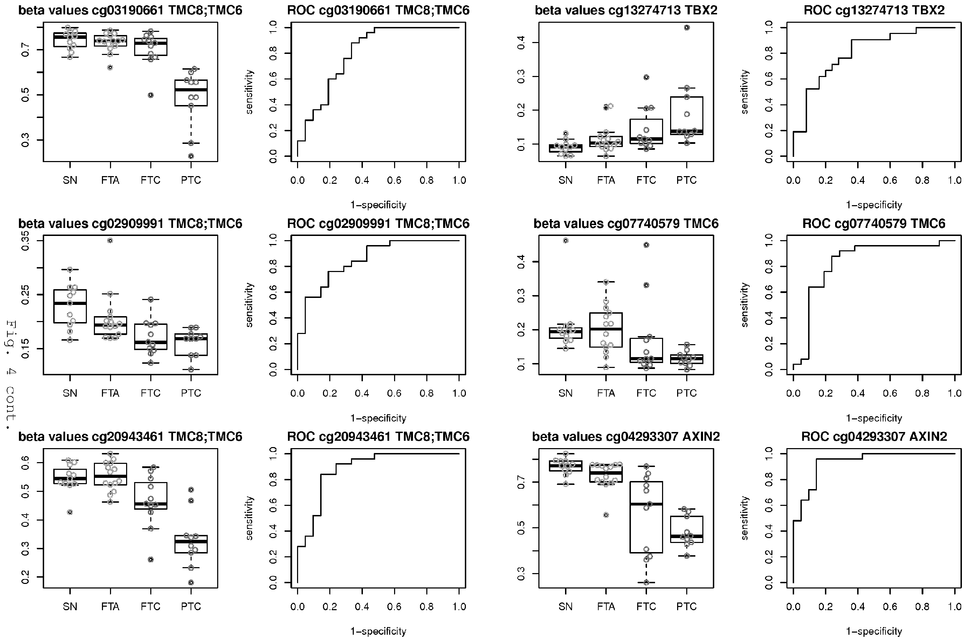

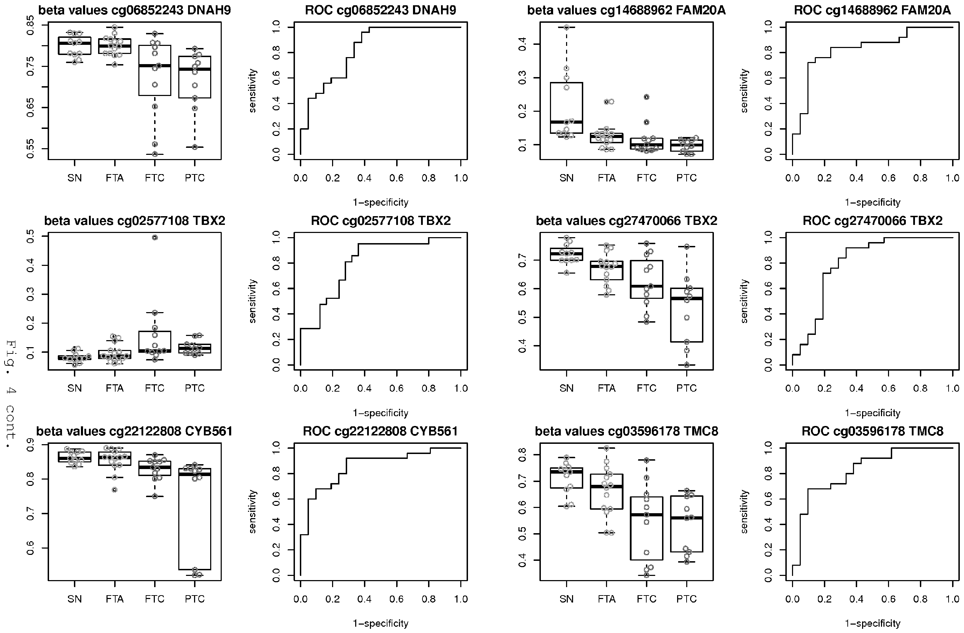

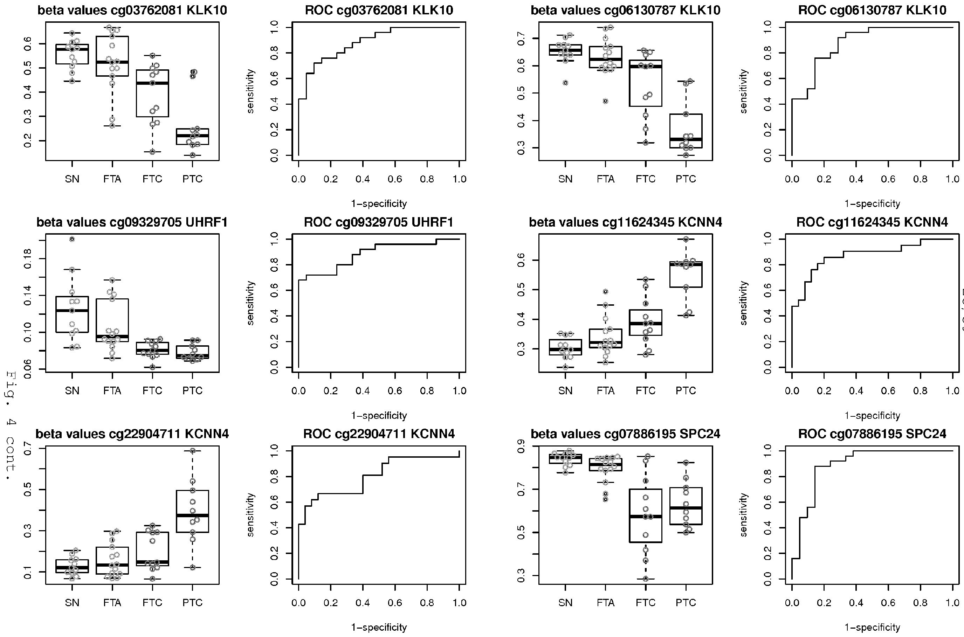

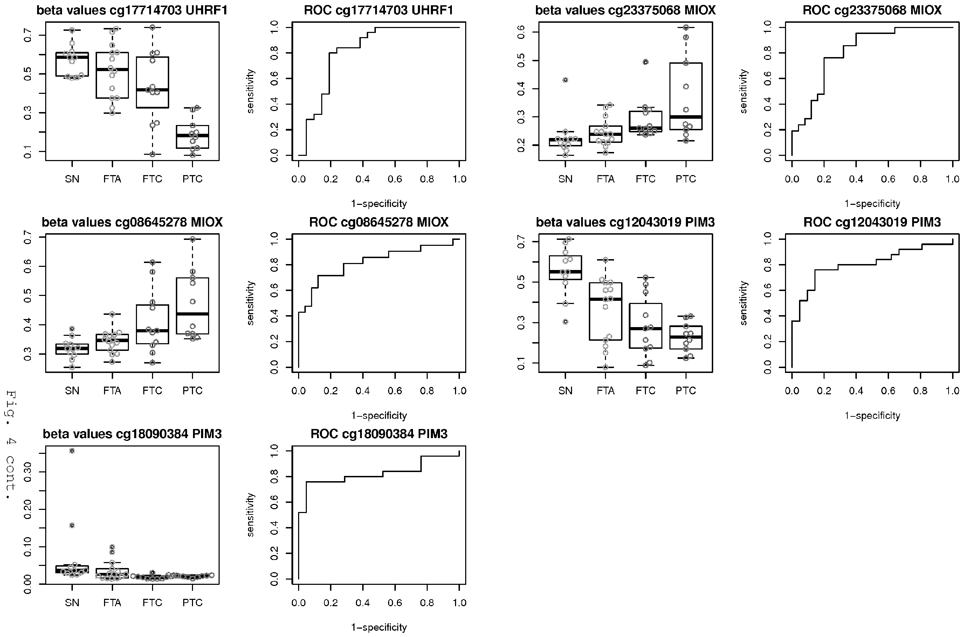

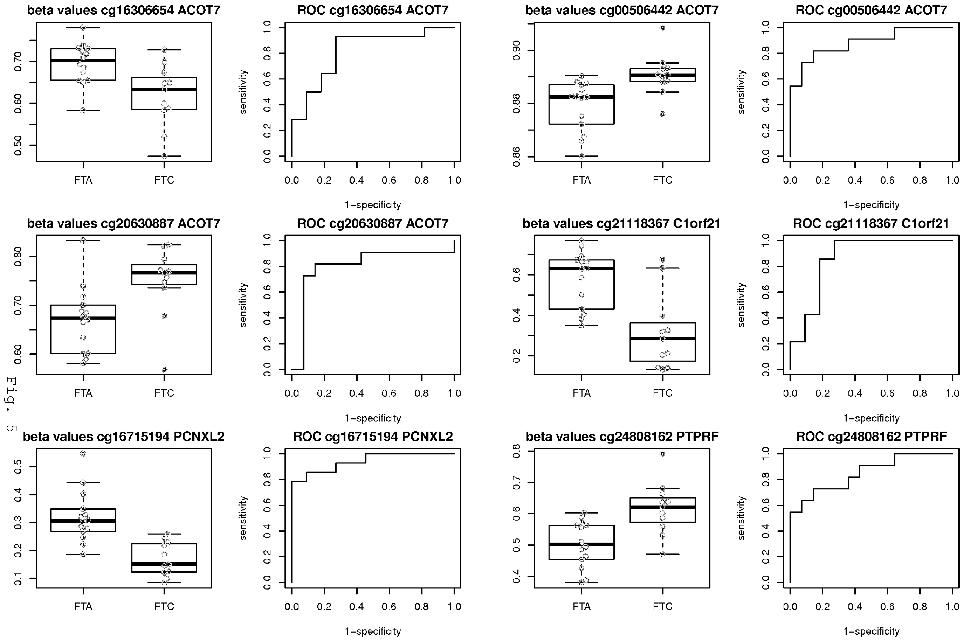

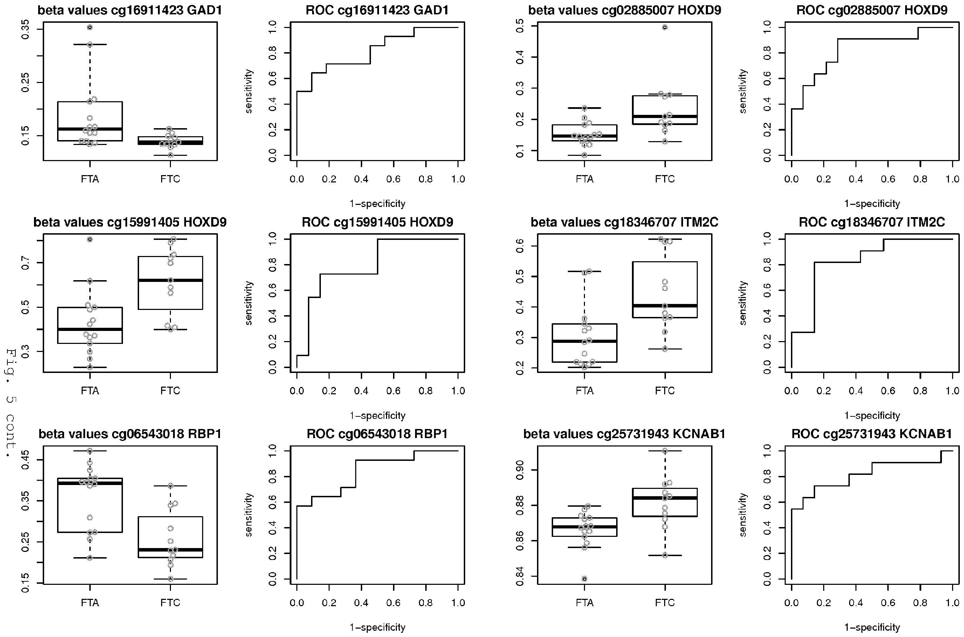

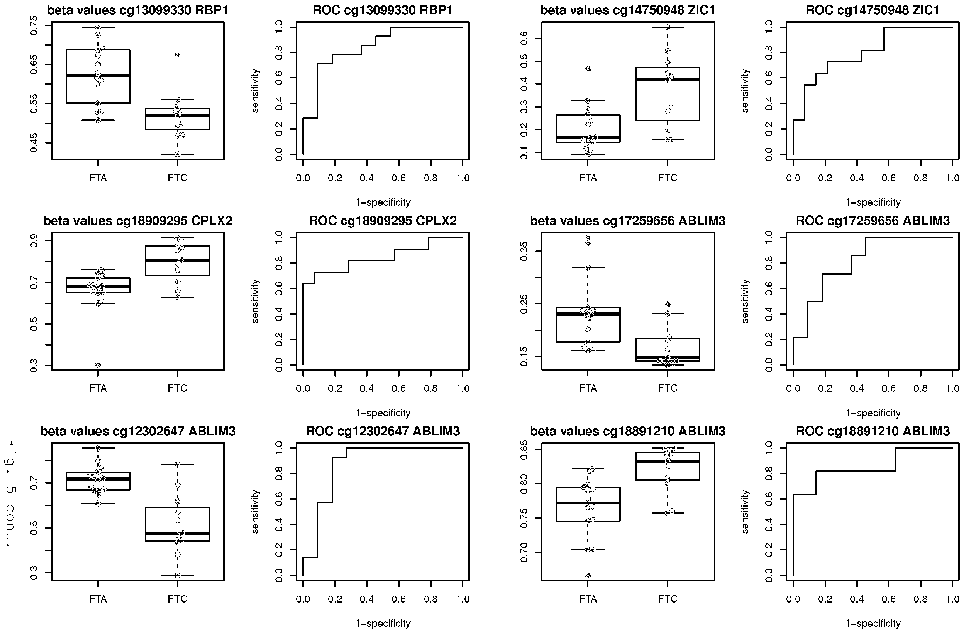

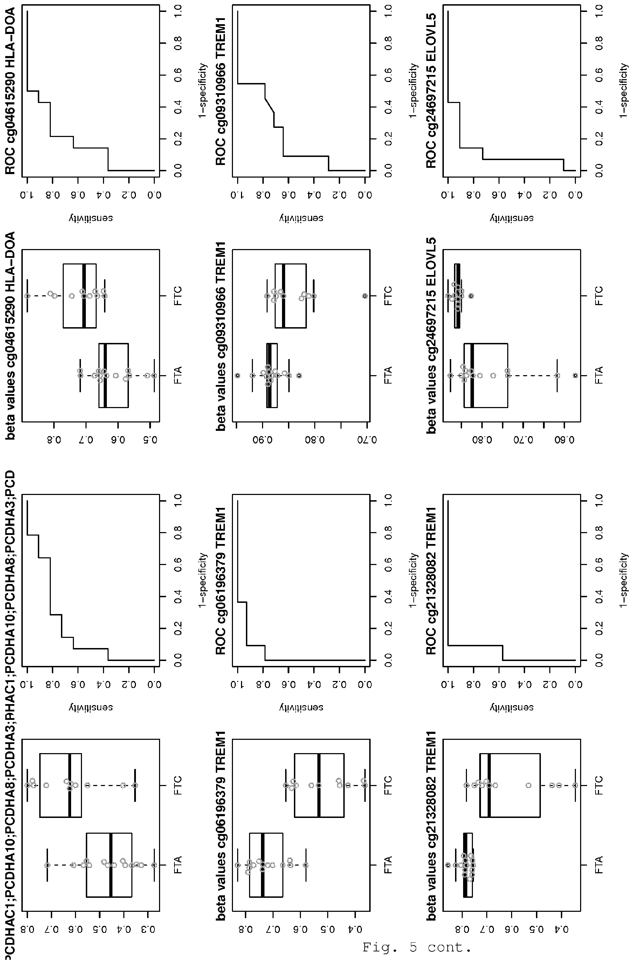

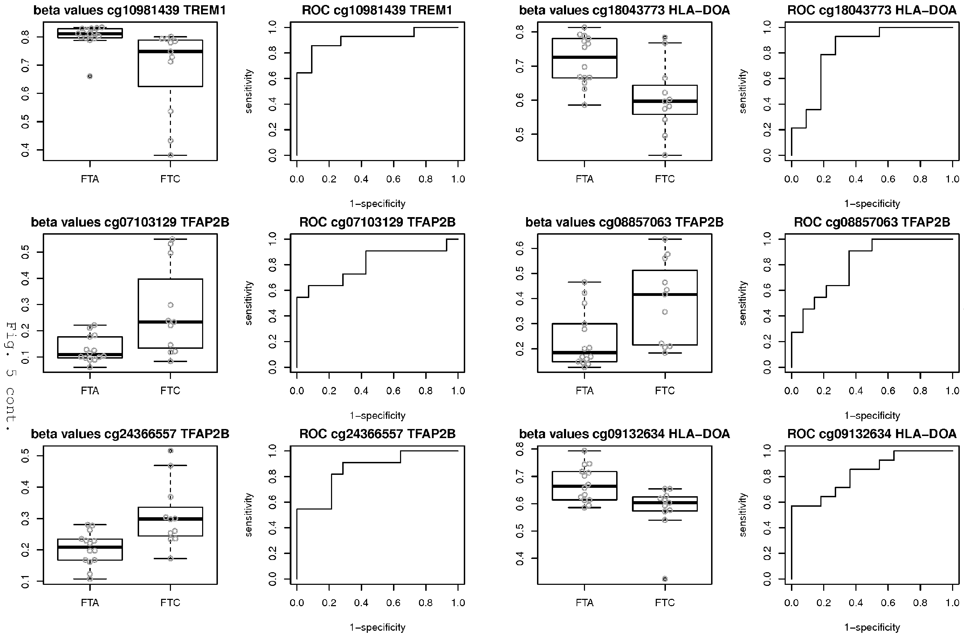

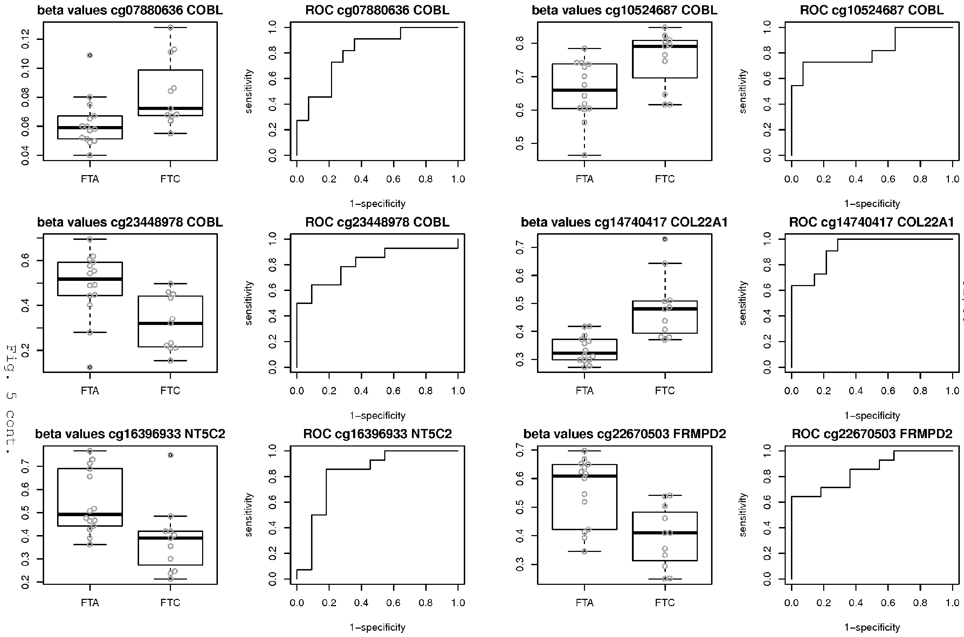

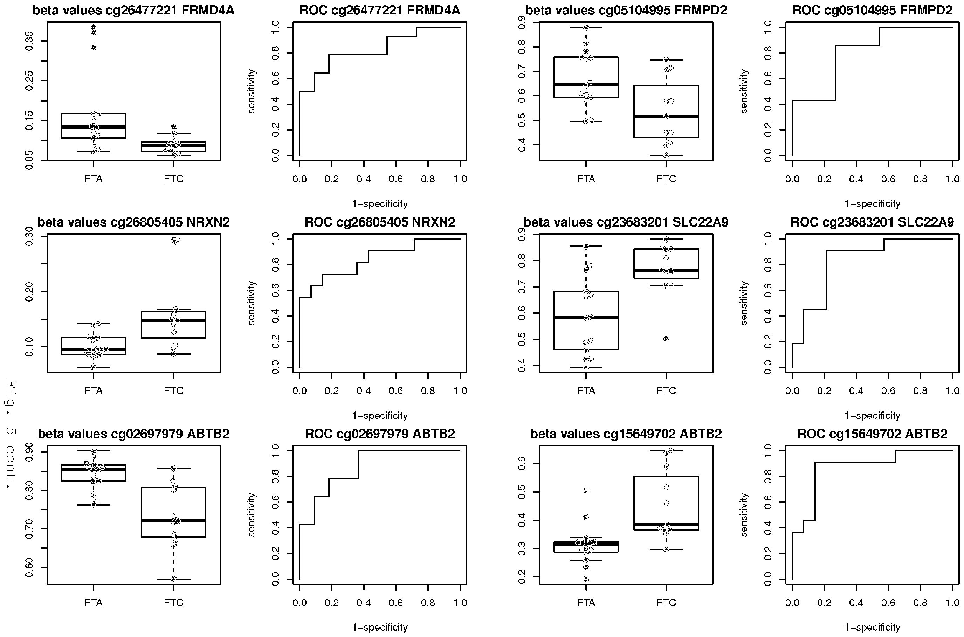

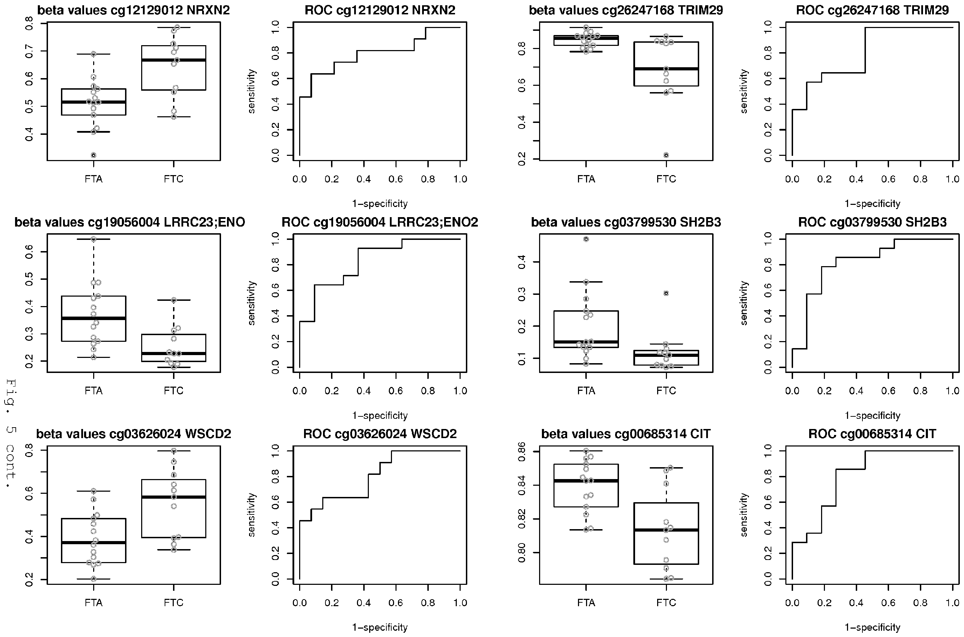

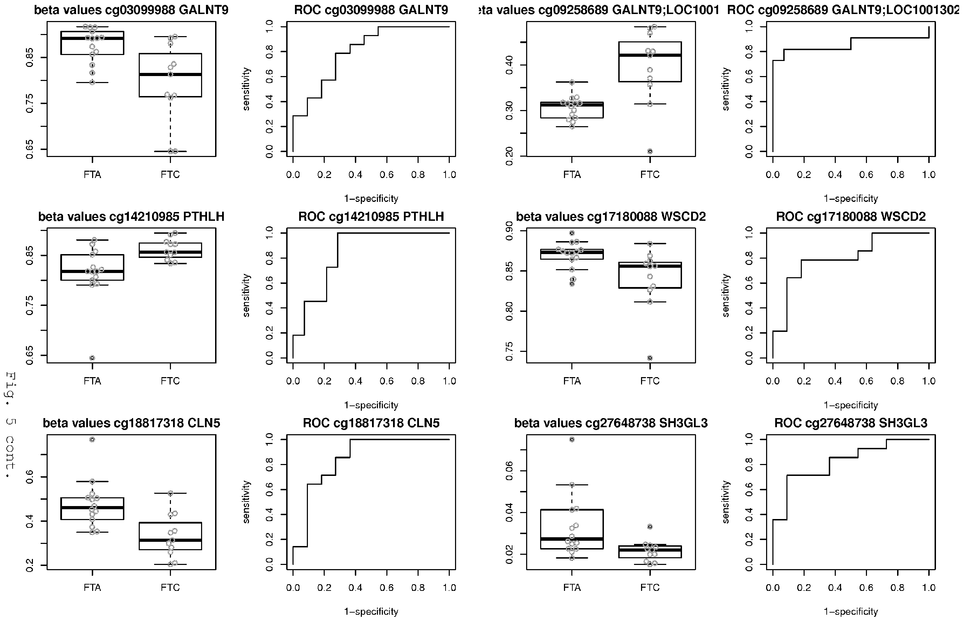

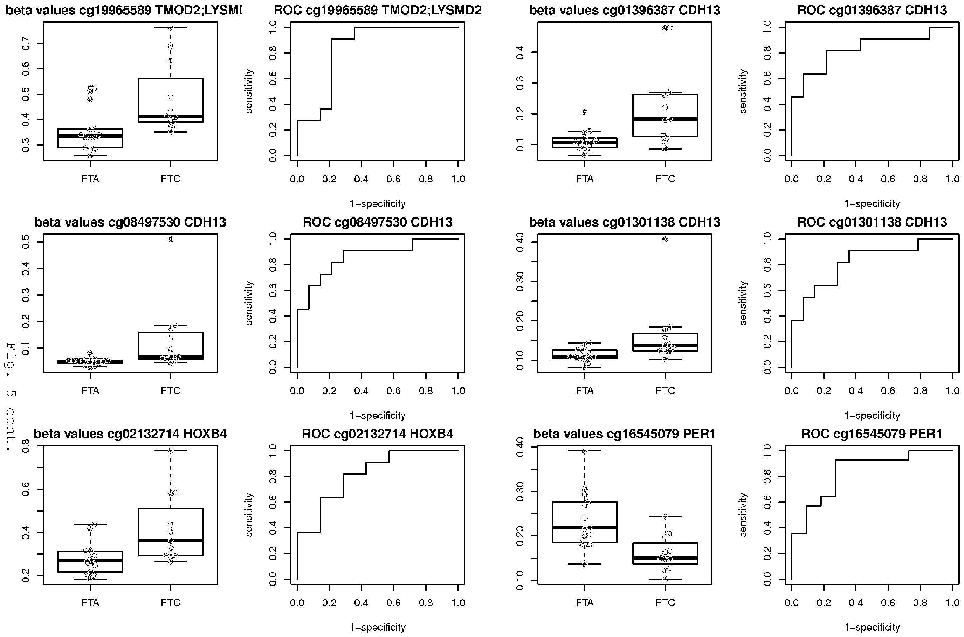

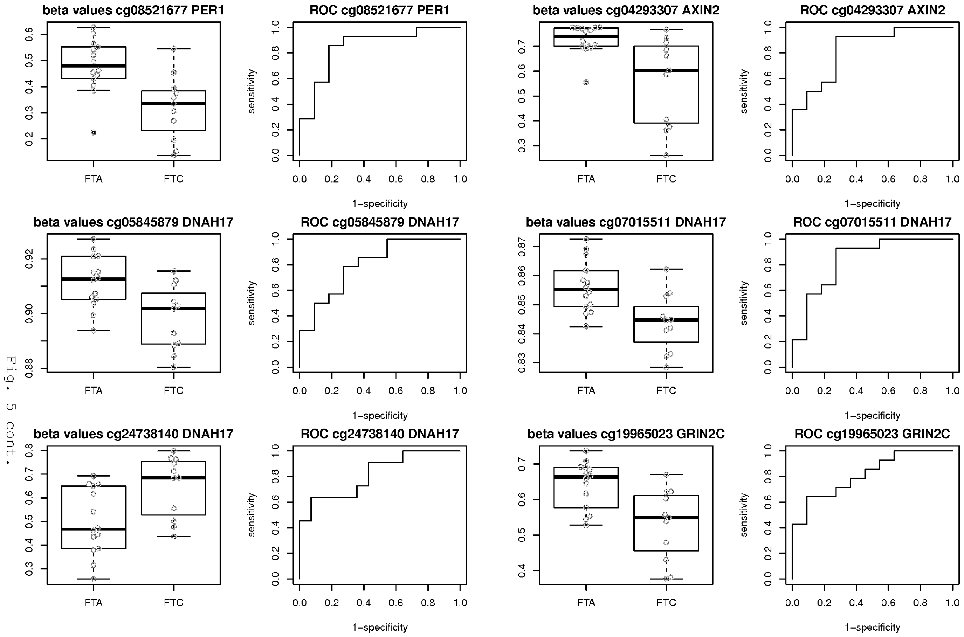

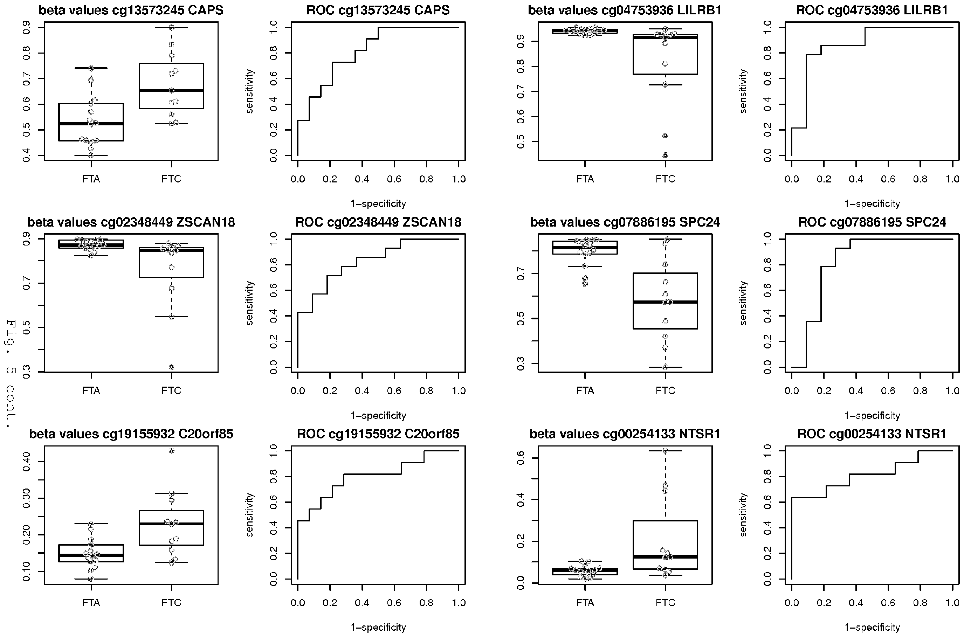

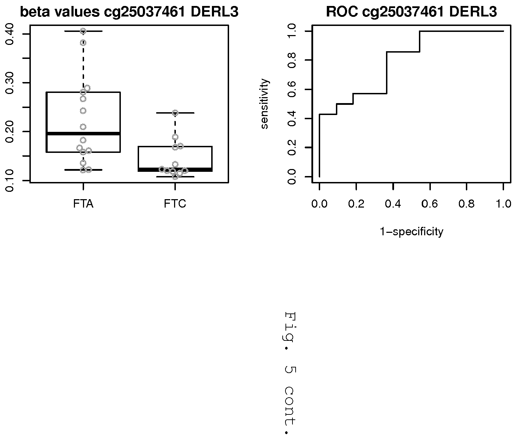

In preferred embodiments, the inventive diagnosis using DNA methylation data is combined with an expression analysis of these genes used in the methylation status analysis or any one of more of the genes of tables 1 and 2, or lists 1a, or 2a. E.g. The method may further comprise determining the gene expression of at least one of said genes of table 1 and/or 2, wherein a differential expression as compared to a normal sample indicates thyroid cancer or the risk thereof. Differential expression may be an increased or decreased expression. Such directions of differential expression are indicated in Figures 4 and 5. The range of levels of differential expression are also indicated in these figures and is e.g. at least 1.5-fold, a least 2-fold, at least 3-fold etc..

The methylation status can be determined by any method known in the art including methylation dependent bisulfite deamination (and consequently the identification of mC - methylated C - changes by any known methods, including PCR and hybridization techniques). Preferably, the methylation status is determined by methylation specific PCR analysis, methylation specific digestion analysis and either or both of hybridisation analysis to non-digested or digested fragments or PCR amplification analysis of non-digested fragments. The methylation status can also be determined by any probes suitable for determining the methylation status including DNA, RNA, PNA, LNA probes which optionally may further include methylation specific moieties.

As further explained below the methylation status can be particularly determined by using hybridisation probes or amplification primer (preferably PCR primers) specific for methylated regions of the inventive marker genes. Discrimination between methylated and non-methylated genes, including the determination of the methylation amount or ratio, can be performed by using e.g. either one of these tools.

The determination using only specific primers aims at specifically amplifying methylated (or in the alternative non-methylated) DNA. This can be facilitated by using (methylation dependent) bisulfite deamination, methylation specific enzymes or by using methylation specific nucleases to digest methylated (or alternatively non-methylated) regions - and consequently only the non-methylated (or alternatively methylated) DNA is obtained. By using a genome chip (or simply a gene chip including hybridization probes for the marker genes), all amplification or non-digested products are detected. I.e. discrimination between methylated and non-methylated states as well as gene selection (the inventive set or subset) is before the step of detection on a chip.

Alternatively it is possible to use universal primers and amplify a multitude of potentially methylated genetic regions (including the genetic markers of the invention) which are, as described either methylation specific amplified or digested, and then use a set of hybridisation probes for the characteristic markers on e.g. a chip for detection. E.g. gene selection is performed on the chip.

Either set, a set of probes or a set of primers, can be used to obtain the relevant methylation data of the genes of the present invention. Of course, both sets can be used.

The method according to the present invention may be performed by any method suitable for the detection of methylation of the marker genes. In order to provide a robust and optionally re-useable test format, the determination of the gene methylation is preferably performed with a DNA-chip, real-time PCR, or a combination thereof. The DNA chip can be a commercially available general gene chip (also comprising a number of spots for the detection of genes not related to the present method) or a chip specifically designed for the method according to the present invention (which predominantly comprises marker gene detection spots).

Preferably the methylated DNA of the sample is detected by a multiplexed hybridization reaction. In further embodiments a methylated DNA is preamplified prior to hybridization, preferably also prior to methylation specific amplification, or digestion. Preferably, also the amplification reaction is multiplexed (e.g. multiplex PCR).

Preferred DNA methylation analyses use bisulfite deamination-based methylation detection or methylation sensitive restriction enzymes. Preferably the restriction enzyme-based strategy is used for elucidation of DNA methylation changes. Further methods to determine methylated DNA are e.g. given in EP 1 369 493 A1 or US 6,605,432. Combining restriction digestion and multiplex PCR amplification with a targeted microarray-hybridization is a particular advantageous strategy to perform the inventive methylation test using the inventive markers. A microarray-hybridization step can be used for reading out the PCR results. For the analysis of the hybridization data statistical approaches for class comparisons and class prediction can be used.

The inventive methods (for the screening of subsets or for diagnosis or prognosis of a disease or tumor type) are particularly suitable to detect low amounts of methylated DNA of the inventive marker genes. Preferably the DNA amount in the sample is below 500ng, below 400ng, below 300ng, below 200ng, below 100ng, below 50ng or even below 25ng. The inventive method is particularly suitable to detect low concentrations of methylated DNA of the inventive marker genes. Preferably the DNA amount in the sample is below 500ng, below 400ng, below 300ng, below 200ng, below 100ng, below 50ng or even below 25ng, per ml sample.

The inventive method may comprise comparing the methylation status with the status of a confirmed thyroid cancer or thyroid cancer type positive and/or negative state. The control may be of a healthy subject or devoid of significant cancer signatures, such as healthy tissue of a healthy subject or SN or FTA.

In particular preferred a negative control is used. The inventive diagnosis may be based on increased methylation of the inventive marker genes. In comparison with other controls a decreased methylation may be detected. Markers with increased or increased methylation in case of cancer or any given thyroid type are shown in tables 1 and 2. The invention may comprise the step of comparing the methylation status with the status of a confirmed thyroid cancer positive and/or negative state, preferably selected from a normal control, FTA, FTC and PTC, preferably wherein the control comprises a healthy thyroid nodule or no nodule.

A particular benefit is surprisingly the use of more than one probe or primer (or primer pair) for each gene, e.g. determining the methylation status for more than one marker, such as CpG sites, islands or shores, of one gene improves the classification rate, despite that the expression level of the same gene is influenced. Thus in preferred embodiments the method comprises determining the methylation status for at least 1, 2, 3, 4, 5, 6, 7, 8, 9, 10, 11, 12 or more genes in at least two (e.g. 2, 3 or more) potentially methylated regions of each gene. These genes may be the ones selected as discussed above of tables 1 and 2. For the inventive set this means that at least 2 probes or primers are included for the mentioned gene(s).

Preferably determining the methylation status comprises comparing a methylation-status specific signal with a methylation-status unspecific signal at a preselected potentially methylated region of said gene. In such embodiments, the inventive methylation status determinations may include generating a signal of a methylation specific probe, i.e. a probe that causes a different signal in dependence of the methylation status, and a methylation status indifferent probe, i.e. a probe, which does not distinguish between the methylation status - also referred to as "methylation unspecific". The ratio of the signal of the methylation specific probe to the signal of the methylation indifferent probe can be used as an indicator of the methylation status of a target nucleic acid. This ratio is also referred to as "beta difference". Using such a ratio has the benefit of normalizing the signal data and cancellation of noise and unwanted signal interferences, that are similar for the methylation specific probe and methylation indifferent probe. Of course this embodiment is not limited to probes but equally applies to any other means of generating methylation dependent and methylation indifferent signal from a target nucleic acids, such as when using primer extension reactions, such as PCR.

The sample of the subject can be a thyroid tissue sample, preferably of a biopsy sample, especially needle aspiration sample. The control sample may be selected from the same type.

In preferred embodiments of the invention, combinable with any one of the other embodiments and gene selections mentioned above, the methylation status of said genes is determined in an upstream region of the open reading frame of the marker genes, in particular a promoter region. In addition or alternatively, it may be determined in a) a nucleic acid defined by the chromosomal locus as identified in table 1 or table 2; b) a CpG site encompassing the nucleic acid a), or c) a one or more nucleic acids within at most 1000 nucleotides in length distanced from said nucleic acid a). The one or more nucleic acid that is preferably determined according to the invention is given by reference to the chromosomal locus (column *MAPINFO* in tables 1 and 2), which together with the chromosome number (column *CHR*) refers to the hg19 human genome assembly (version "GRCh/hg19" of Feb. 2009 - see http://genome-euro.ucsc.edu) and identifies an exact position in the genome by a single base). A further preferred nucleic acid or CpG locus for detection may be within the vicinity of the more preferred nucleic acid locus that includes the position of the chromosomal locus as identified in table 1 or table 2, e.g. within at most 800, at most 600, at most 500, at most 400, at most 300, at most 200, or at most 100, nucleotides in length distanced from said nucleic acid a).

In a further aspect, the present invention provides a set of nucleic acid primers, primer pairs or hybridization probes being specific for a potentially methylated region of marker genes being suitable to diagnose or predict thyroid cancer according to any method of the invention, E.g. the set may comprise probes or primers or primer pairs for genes ABLIM3, ABTB2, ACOT7, ADM, ALOX5, ANKRD22, AXIN2, BHLHE40, C10orf107, C1orf21, C20orf85, CAPS, CDH13, CHKA, CIITA, CIT, CLN5, COBL, COL22A1, CPLX2, CYB561, DERL3, DNAH17, DNAH9, ELMO1, ELOVL5, ENO2, EPHA10, FAM20A, FMOD, FRMD4A, FRMPD2, GAD1, GALNT9, GJB6, GRIN2C, HK1, HLA-DOA, HOXB4, HOXD9, IFT140, IL17RD, IP6K3, IRF5, ITM2C, ITPR1, KCNAB1, KCNN4, KLK10, KRT80, LILRB1, LIPH, LOC100130238, LRP2, LRRC23, LYSMD2, MACC1, MICALCL, MINA, MIOX, MPPED2, MTSS1, MYO1G, NEK11, NRXN2, NT5C2, NTSR1, PAG1, PCDHA, PCNXL2, PCNXL2, PDZK1IP1, PDZRN4, PERI, PIM3, PRDM11, PRR7, PTHLH, PTPRF, RBP1, RUNX2, SH2B3, SH3GL3, SLC22A9, SORBS2, SPC24, STRA6, SUPT3H, SYN2, TBX2, TFAP2B, TIMP4, TMC6, TMC8, TMEM204, TMOD2, TREM1, TRIM29, UHRF1, WSCD2, ZIC1, ZSCAN18. Preferably at least 3 probes and/or primers for genes selected from three or more of the genes of table 1 and/or table 2, are selected. Preferably at least one thyroid cancer gene is selected from ABTB2, ACOT7, ADM, ALOX5, ANKRD22, AXIN2, BHLHE40, C10orf107, C1orf21, C20orf85, CAPS, CHKA, CIITA, CIT, CLN5, COBL, COL22A1, CPLX2, DERL3, DNAH17, DNAH9, ELMO1, ELOVL5, ENO2, FAM20A, FMOD, FRMPD2, GALNT9, GJB6, GRIN2C, HK1, HLA-DOA, HOXD9, IFT140, IL17RD, IP6K3, ITM2C, ITPR1, KCNAB1, KCNN4, KRT80, LILRB1, LIPH, LOC100130238, LRP2, LRRC23, LYSMD2, MACC1, MICALCL, MINA, MPPED2, MTSS1, MYO1G, NEK11, NRXN2, NT5C2, NTSR1, PAG1, a PCDHA other than PCDHA13, PCNXL2, PCNXL2, PDZK1IP1, PDZRN4, PERI, PIM3, PRDM11, PRR7, PTHLH, PTPRF, RUNX2, SH2B3, SH3GL3, SLC22A9, SORBS2, SPC24, SUPT3H, SYN2, TFAP2B, TIMP4, TMC6, TMC8, TMEM204, TMOD2, TREM1, TRIM29, UHRF1, WSCD2, ZSCAN18. Also preferred, the set contains at most 5000 probes or primers (or any maximum number given above).

Preferably, the primer pairs and probes are specific for a methylated upstream region of the open reading frame of the marker genes, in particular a promoter region; or specific for a) a nucleic acid defined by the chromosomal locus as identified in table 1 or table 2; b) a CpG site encompassing the nucleic acid a), or c) a nucleic acid within at most 1000 nucleotides in length distanced from said nucleic acid a). Preferably as further defines as above.

Preferably, the set further comprises probes or primer specific for the potentially specific for a potentially methylated region of marker genes, wherein said further probes or primers are non-specific for DNA methylation and are suitable for use as a control or normalization agent. Also, such methylation unspecific probes can be used to determine a beta difference as disclosed above. The inventive set may also comprise a computer readable memory device, such as a CD, DVD, BR, flash drive, with a computer program product for calculating such normalizations or, in general, for assisting in a method of the invention, including the statistical methods described above.

Set according to the invention may be provided in a kit together with a methylation specific restriction enzyme and/or a reagent for bisulfite nucleotide deamination; and/or wherein the set comprises probes on a microarray.

Preferably the set is provided on a solid surface, in particular a chip, whereon the primers or probes can be immobilized. Solid surfaces or chips may be of any material suitable for the immobilization of biomolecules such as the moieties, including glass, modified glass (aldehyde modified) or metal chips.

The primers or probes can also be provided as such, including lyophilized forms or being in solution, preferably with suitable buffers. The probes and primers can of course be provided in a suitable container, e.g. a tube or micro tube.

The inventive marker set, including certain disclosed subsets, which can be identified with the methods disclosed herein, are suitable to distinguish between thyroid cancer, SN, FTA, FTC and PTC, in particular for diagnostic or prognostic uses.

The present invention is further explained by way of the following figures and examples, without being limited to these embodiments of the invention. The invention as described above can of course be combined with any element of these examples.

### Tables

**Table 1: 126 CpG sites which map to 63 genes and distinguish benign vs. malignant (Probe... probe identification number, Chr... chromosome number, mapinfo... chromosome position, AUC... area under curve)**

| **PROBE** | **CHR** | **MAPINFO** | **GENE SYMBOL** | **AUC** | **error rate** | **P-Value** | **Beta.Difference** |
|---|---|---|---|---|---|---|---|
| cgl7259656 | 5 | 148521112 | ABLIM3 | 0,832 | 0,457 | 3,03E-04 | -0,071 |
| cg02995045 | 1 | 6419906 | ACOT7 | 0,846 | 0,283 | 1,74E-05 | -0,180 |
| cg16306654 | 1 | 6419767 | ACOT7 | 0,893 | 0,457 | 1,57E-06 | -0,099 |
| cg00506442 | 1 | 6340054 | ACOT7 | 0,811 | 0,457 | 1,04E-03 | 0,010 |
| cg20630887 | 1 | 6417823 | ACOT7 | 0,817 | 0,457 | 3,76E-04 | 0,091 |
| cgl0044466 | 11 | 10328911 | ADM | 0,830 | 0,239 | 1,89E-05 | -0,207 |
| cg06875754 | 11 | 10328428 | ADM | 0,876 | 0,283 | 5,52E-06 | -0,168 |
| cg23084016 | 10 | 45916904 | ALOX5 | 0,806 | 0,457 | 1,76E-04 | -0,091 |
| cg24065504 | 10 | 90613015 | ANKRD22 | 0,838 | 0,326 | 5,43E-05 | -0,173 |
| cg03249630 | 10 | 90611782 | ANKRD22 | 0,829 | 0,457 | 4,43E-04 | -0,093 |
| cg04293307 | 17 | 63553581 | AXIN2 | 0,933 | 0,217 | 2,18E-09 | -0,222 |
| cg20971407 | 3 | 5022392 | BHLHE40 | 0,861 | 0,196 | 1,78E-06 | -0,220 |
| cg16582517 | 3 | 5025885 | BHLHE40 | 0,869 | 0,196 | 1,53E-07 | -0,247 |
| cg16320419 | 3 | 5025570 | BHLHE40 | 0,808 | 0,348 | 2,96E-04 | -0,136 |
| cg01180628 | 3 | 5023394 | BHLHE40 | 0,808 | 0,413 | 6,96E-04 | -0,161 |
| cg04764597 | 10 | 63510947 | C10orf107 | 0,804 | 0,413 | 8,43E-04 | -0,115 |
| cg21118367 | 1 | 184460875 | C1orf21 | 0,916 | 0,196 | 2,80E-09 | -0,314 |
| cg00172631 | 1 | 184435459 | C1orf21 | 0,834 | 0,348 | 6,72E-05 | 0,136 |
| cg17556527 | 11 | 67859023 | CHKA | 0,829 | 0,457 | 8,78E-04 | 0,048 |
| cg01105356 | 16 | 11016097 | CIITA | 0,808 | 0,478 | 2,38E-03 | -0,106 |
| cg00685314 | 12 | 120307689 | CIT | 0,851 | 0,457 | 5,78E-05 | -0,058 |
| cg03339668 | 12 | 120241957 | CIT | 0,802 | 0,457 | 1,69E-03 | -0,082 |
| cg23448978 | 7 | 51209365 | COBL | 0,846 | 0,196 | 6,06E-06 | -0,181 |
| cg14525527 | 7 | 51096783 | COBL | 0,827 | 0,457 | 3,21E-04 | 0,028 |
| cg27590143 | 7 | 51175394 | COBL | 0,808 | 0,457 | 3,97E-01 | 0,013 |
| cg22122808 | 17 | 61511683 | CYB561 | 0,859 | 0,457 | 1,95E-03 | -0,074 |
| cg03464847 | 17 | 11501580 | DNAH9 | 0,859 | 0,457 | 2,10E-05 | 0,038 |
| cg06852243 | 17 | 11505169 | DNAH9 | 0,832 | 0,457 | 7,88E-05 | -0,078 |
| cg24237862 | 7 | 37026842 | ELMO1 | 0,838 | 0,304 | 4,67E-06 | -0,195 |
| cg04622024 | 1 | 38201001 | EPHA10 | 0,842 | 0,391 | 1,51E-05 | 0,127 |
| cg24375409 | 1 | 38200920 | EPHA10 | 0,821 | 0,391 | 4,43E-04 | 0,138 |
| cg11664987 | 1 | 38201123 | EPHA10 | 0,842 | 0,457 | 1,94E-04 | 0,075 |
| cg15761609 | 17 | 66598067 | FAM20A | 0,821 | 0,304 | 6,55E-05 | -0,168 |
| cg14688962 | 17 | 66596275 | FAM20A | 0,829 | 0,457 | 4,66E-03 | -0,060 |
| cg26894354 | 1 | 203311314 | FMOD | 0,811 | 0,413 | 6,72E-04 | -0,120 |
| cg09203312 | 13 | 20805196 | GJB6 | 0,804 | 0,522 | 4,47E-04 | 0,119 |
| cg20372666 | 10 | 71149910 | HK1 | 0,817 | 0,217 | 1,33E-05 | 0,175 |
| cg15358372 | 10 | 71108752 | HK1 | 0,880 | 0,261 | 3,22E-06 | 0,223 |
| cg16001913 | 10 | 71029644 | HK1 | 0,808 | 0,457 | 1,42E-03 | -0,077 |
| cg00078759 | 16 | 1600969 | IFT140;TMEM204 | 0,829 | 0,457 | 5,95E-05 | 0,078 |
| cg00217171 | 16 | 1590847 | IFT140;TMEM204 | 0,863 | 0,457 | 3,63E-05 | 0,034 |
| cg02730055 | 16 | 1600926 | IFT140;TMEM204 | 0,859 | 0,457 | 1,60E-05 | 0,055 |
| cg04391232 | 16 | 1591854 | IFT140;TMEM204 | 0,890 | 0,457 | 4,40E-06 | 0,043 |
| cg05174855 | 16 | 1592091 | IFT140;TMEM204 | 0,819 | 0,457 | 5,95E-05 | 0,037 |
| cg07271253 | 16 | 1591768 | IFT140;TMEM204 | 0,855 | 0,457 | 1,94E-05 | 0,041 |
| cg26596419 | 16 | 1591503 | IFT140;TMEM204 | 0,827 | 0,457 | 1,26E-04 | |
| cg13717817 | 3 | 57177391 | IL17RD | 0,817 | 0,239 | 5,25E-05 | 0,171 |
| cg18257103 | 6 | 33714907 | IP6K3 | 0,878 | 0,261 | 6,40E-07 | 0,153 |
| cg10714061 | 6 | 33714631 | IP6K3 | 0,884 | 0,261 | 7,57E-07 | 0,193 |
| cg00140447 | 7 | 128580709 | IRF5 | 0,817 | 0,413 | 1,78E-03 | -0,107 |
| cg04864179 | 7 | 128579964 | IRF5 | 0,861 | 0,457 | 5,21E-03 | -0,046 |
| cg05904013 | 7 | 128579933 | IRF5 | 0,821 | 0,457 | 7,90E-03 | -0,050 |
| cg24126180 | 7 | 128580582 | IRF5 | 0,823 | 0,457 | 1,34E-02 | -0,069 |
| cg12320198 | 3 | 4557437 | ITPR1 | 0,825 | 0,304 | 5,37E-05 | 0,163 |
| cg26395694 | 3 | 4783306 | ITPR1 | 0,834 | 0,391 | 2,75E-05 | -0,143 |
| cg11382241 | 3 | 4889445 | ITPR1 | 0,821 | 0,457 | 7,47E-05 | 0,088 |
| cg21407899 | 3 | 4867340 | ITPR1 | 0,842 | 0,478 | 1,19E-05 | -0,135 |
| cg23662097 | 3 | 4873008 | ITPR1 | 0,832 | 0,543 | 7,66E-05 | -0,146 |
| cg03341748 | 3 | 156091058 | KCNAB1 | 0,842 | 0,370 | 1,36E-05 | 0,164 |
| cg11624345 | 19 | 44278551 | KCNN4 | 0,872 | 0,261 | 1,30E-06 | 0,145 |
| cg22904711 | 19 | 44278628 | KCNN4 | 0,804 | 0,348 | 1,15E-04 | 0,147 |
| cg03762081 | 19 | 51523565 | KLK10 | 0,886 | 0,217 | 4,96E-07 | -0,206 |
| cg06130787 | 19 | 51523550 | KLK10 | 0,882 | 0,239 | 1,10E-07 | -0,187 |
| cg07925587 | 12 | 52583324 | KRT80 | 0,863 | 0,304 | 2,33E-06 | -0,163 |
| cg11051139 | 12 | 52580428 | KRT80 | 0,848 | 0,457 | 1,65E-04 | -0,033 |
| cg23243343 | 12 | 52579609 | KRT80 | 0,848 | 0,457 | 6,77E-04 | -0,079 |
| cg24506604 | 12 | 52579502 | KRT80 | 0,834 | 0,457 | 3,47E-03 | -0,061 |
| cg04472592 | 12 | 52585786 | KRT80 | 0,802 | 0,478 | 3,20E-04 | -0,118 |
| cg02124892 | 3 | 185270360 | LIPH | 0,876 | 0,217 | 9,17E-07 | -0,199 |
| cg08099797 | 3 | 185270308 | LIPH | 0,924 | 0,261 | 2,13E-08 | -0,222 |
| cg12611448 | 3 | 185255217 | LIPH | 0,850 | 0,348 | 3,88E-05 | -0,148 |
| cg23620049 | 3 | 185270558 | LIPH | 0,889 | 0,413 | 5,29E-05 | -0,135 |
| cg02361027 | 2 | 170217401 | LRP2 | 0,836 | 0,239 | 5,84E-06 | 0,246 |
| cg12424504 | 7 | 20179965 | MACC1 | 0,844 | 0,196 | 1,72E-06 | 0,215 |
| cg26158270 | 11 | 12309622 | MICALCL | 0,857 | 0,391 | 2,83E-05 | -0,125 |
| cg19850728 | 3 | 97688465 | MINA | 0,823 | 0,304 | 9,01E-05 | 0,147 |
| cg08645278 | 22 | 50925232 | MIOX | 0,821 | 0,435 | 8,11E-05 | 0,106 |
| cg23375068 | 22 | 50925113 | MIOX | 0,813 | 0,457 | 1,58E-03 | 0,090 |
| cg01438090 | 11 | 30502936 | MPPED2 | 0,931 | 0,174 | 1,59E-08 | 0,228 |
| cg05026393 | 8 | 1256727 95 | MTSS1 | 0,870 | 0,500 | 5,23E-06 | -0,122 |
| cg22111043 | 7 | 45019005 | MYO1G | 0,890 | 0,370 | 3,09E-05 | -0,154 |
| cg06787669 | 7 | 45018789 | MYO1G | 0,848 | 0,370 | 4,87E-05 | -0,159 |
| cg10673833 | 7 | 45018849 | MYO1G | 0,821 | 0,370 | 1,21E-04 | -0,145 |
| cg21188037 | 7 | 45018658 | MYO1G | 0,811 | 0,370 | 4,86E-04 | -0,143 |
| cg06239593 | 3 | 130748639 | NEK11 | 0,880 | 0,239 | 1,14E-06 | 0,229 |
| cg09973676 | 8 | 82006417 | PAG1 | 0,855 | 0,370 | 4,19E-05 | -0,141 |
| cg16715194 | 1 | 233430825 | PCNXL2 | 0,893 | 0,239 | 9,31E-07 | -0,165 |
| cg09258479 | 1 | 47655861 | PDZK1IP1 | 0,914 | 0,152 | 1,24E-08 | -0,183 |
| cg02291556 | 1 | 47656140 | PDZK1IP1 | 0,901 | 0,174 | 5,10E-08 | -0,202 |
| cg06619077 | 1 | 47656003 | PDZK1IP1 | 0,851 | 0,174 | 2,10E-05 | -0,191 |
| cg07150145 | 1 | 47656137 | PDZK1IP1 | 0,939 | 0,239 | 9,60E-09 | -0,169 |
| cg07810156 | 1 | 47655682 | PDZK1IP1 | 0,817 | 0,457 | 3,44E-05 | -0,049 |
| cg05992726 | 12 | 41967396 | PDZRN4 | 0,825 | 0,457 | 2,94E-03 | 0,064 |
| cg12043019 | 22 | 50356277 | PIM3 | 0,806 | 0,261 | 7,58E-05 | -0,193 |
| cg18090384 | 22 | 50355424 | PIM3 | 0,825 | 0,457 | 3,61E-02 | -0,033 |
| cg27340283 | 11 | 45199222 | PRDM11 | 0,802 | 0,457 | 8,71E-03 | 0,056 |
| cg05648472 | 11 | 45232364 | PRDM11 | 0,811 | 0,478 | 1,43E-03 | -0,085 |
| cg14098951 | 5 | 176875120 | PRR7 | 0,806 | 0,283 | 2,99E-04 | -0,175 |
| cg05217983 | 6 | 45406867 | RUNX2 | 0,840 | 0,457 | 3,29E-04 | -0,086 |
| cg15923139 | 4 | 186801896 | SORBS2 | 0,903 | 0,152 | 1,01E-08 | 0,260 |
| cg17006136 | 4 | 186559412 | SORBS2 | 0,804 | 0,435 | 5,23E-03 | -0,096 |
| cg07886195 | 19 | 11263615 | SPC24 | 0,893 | 0,196 | 5,55E-08 | -0,208 |
| cg21068293 | 15 | 74496576 | STRA6 | 0,846 | 0,457 | 3,90E-05 | -0,066 |
| cg01946401 | 6 | 45296101 | SUPT3H;RUNX2 | 0,802 | 0,457 | 1,54E-04 | -0,105 |
| cg05112986 | 6 | 45346247 | SUPT3H;RUNX2 | 0,861 | 0,457 | 8,67E-06 | -0,054 |
| cgl0110335 | 3 | 12197630 | SYN2;TIMP4 | 0,874 | 0,239 | 1,63E-07 | -0,183 |
| cg27470066 | 17 | 59485779 | TBX2 | 0,802 | 0,435 | 7,44E-05 | -0,112 |
| cg13274713 | 17 | 59477286 | TBX2 | 0,808 | 0,457 | 2,47E-03 | 0,061 |
| cg02577108 | 17 | 59478194 | TBX2 | 0,808 | 0,457 | 9,92E-03 | 0,050 |
| cg07740579 | 17 | 76124173 | TMC6 | 0,830 | 0,457 | 1,41E-02 | -0,061 |
| cg03596178 | 17 | 76138514 | TMC8 | 0,829 | 0,326 | 3,36E-05 | -0,143 |
| cg20943461 | 17 | 76126886 | TMC8;TMC6 | 0,884 | 0,261 | 4,03E-07 | -0,149 |
| cg01246266 | 17 | 76126490 | TMC8;TMC6 | 0,880 | 0,391 | 8,18E-06 | -0,128 |
| cg03190661 | 17 | 76126702 | TMC8;TMC6 | 0,806 | 0,391 | 7,10E-05 | -0,138 |
| cg00447208 | 17 | 76126301 | TMC8;TMC6 | 0,821 | 0,413 | 4,60E-04 | -0,110 |
| cg02909991 | 17 | 76127829 | TMC8;TMC6 | 0,853 | 0,457 | 7,73E-05 | -0,049 |
| cg06196379 | 6 | 41254885 | TREM1 | 0,937 | 0,196 | 7,95E-09 | -0,207 |
| cg21328082 | 6 | 41254471 | TREM1 | 0,981 | 0,239 | 4,94E-09 | -0,221 |
| cg10981439 | 6 | 41254433 | TREM1 | 0,930 | 0,348 | 2,35E-05 | -0,160 |
| cg09310966 | 6 | 41254825 | TREM1 | 0,893 | 0,457 | 2,15E-05 | -0,077 |
| cg17714703 | 19 | 4912221 | UHRF1 | 0,823 | 0,217 | 1,36E-05 | -0,224 |
| cg09329705 | 19 | 4909474 | UHRF1 | 0,884 | 0,457 | 1,93E-05 | -0,035 |
| cg03626024 | 12 | 108524345 | WSCD2 | 0,857 | 0,217 | 7,93E-06 | 0,187 |
| cg00770443 | 12 | 108611845 | WSCD2 | 0,888 | 0,457 | 1,81E-07 | -0,076 |
| cg17180088 | 12 | 108629501 | WSCD2 | 0,815 | 0,457 | 2,59E-04 | -0,027 |
| cg00736201 | 12 | 108643267 | WSCD2 | 0,872 | 0,457 | 6,65E-06 | -0,046 |

**Table 2: 73 CpG sites which map to 65 genes and distinguish FTA vs. FTC (abbreviations as in table 1)**

| **PROBE** | **CHR** | **MAPINFO** | **GENE SYMBOL** | **AUC** | **error rate** | **P-Value** | **Beta.Difference** |
|---|---|---|---|---|---|---|---|
| cg00506442 | 1 | 6340054 | ACOT7 | 0,883 | 0,44 | 6,18E-03 | 0,012 |
| cg20630887 | 1 | 6417823 | ACOT7 | 0,805 | 0,44 | 8,80E-03 | 0,079 |
| cg16306654 | 1 | 6419767 | ACOT7 | 0,821 | 0,44 | 4,42E-03 | -0,077 |
| cg24808162 | 1 | 44067587 | PTPRF | 0,854 | 0,44 | 1,28E-03 | 0,113 |
| cg21118367 | 1 | 184460875 | C1orf21 | 0,864 | 0,2 | 3,71E-04 | -0,265 |
| cg16715194 | 1 | 233430825 | PCNXL2 | 0,942 | 0,44 | 8,20E-05 | -0,151 |
| cg16911423 | 2 | 171673866 | GAD1 | 0,815 | 0,44 | 2,57E-02 | -0,049 |
| cg02885007 | 2 | 176987605 | HOXD9 | 0,834 | 0,44 | 6,46E-03 | 0,084 |
| cg15991405 | 2 | 176988480 | HOXD9 | 0,805 | 0,52 | 5,18E-03 | 0,183 |
| cg18346707 | 2 | 231732249 | ITM2C | 0,831 | 0,44 | 4,88E-03 | 0,139 |
| cg13099330 | 3 | 139257799 | RBP1 | 0,851 | 0,44 | 1,05E-03 | -0,105 |
| cg06543018 | 3 | 139258822 | RBP1 | 0,844 | 0,44 | 2,38E-03 | -0,102 |
| cg14750948 | 3 | 147130477 | ZIC1 | 0,805 | 0,48 | 4,68E-03 | 0,163 |
| cg25731943 | 3 | 156252078 | KCNAB1 | 0,821 | 0,44 | 7,66E-03 | 0,015 |
| cg25487047 | 5 | 140389945 | PCDHA | 0,812 | 0,48 | 5,64E-03 | 0,160 |
| cg17259656 | 5 | 148521112 | ABLIM3 | 0,815 | 0,44 | 6,36E-03 | -0,069 |
| cg12302647 | 5 | 148533875 | ABLIM3 | 0,877 | 0,4 | 7,21E-05 | -0,198 |
| cg18891210 | 5 | 148560634 | ABLIM3 | 0,857 | 0,44 | 2,12E-03 | 0,056 |
| cg18909295 | 5 | 175223293 | CPLX2 | 0,844 | 0,44 | 2,51E-03 | 0,142 |
| cg09132634 | 6 | 32974122 | HLA-DOA | 0,831 | 0,44 | 6,07E-03 | -0,092 |
| cg18043773 | 6 | 32974906 | HLA-DOA | 0,828 | 0,44 | 3,77E-03 | -0,110 |
| cg04615290 | 6 | 32978129 | HLA-DOA | 0,834 | 0,44 | 1,41E-03 | 0,105 |
| cgl0981439 | 6 | 41254433 | TREM1 | 0,906 | 0,44 | 8,57E-03 | -0,121 |
| cg21328082 | 6 | 41254471 | TREM1 | 0,961 | 0,44 | 4,00E-04 | -0,169 |
| cg09310966 | 6 | 41254825 | TREM1 | 0,805 | 0,44 | 8,95E-03 | -0,047 |
| cg06196379 | 6 | 41254885 | TREM1 | 0,964 | 0,36 | 4,63E-06 | -0,206 |
| cg24366557 | 6 | 50787650 | TFAP2B | 0,857 | 0,44 | 2,40E-03 | 0,106 |
| cg07103129 | 6 | 50787964 | TFAP2B | 0,802 | 0,44 | 4,51E-03 | 0,148 |
| cg08857063 | 6 | 50808667 | TFAP2B | 0,815 | 0,48 | 1,07E-02 | 0,151 |
| cg24697215 | 6 | 53185643 | ELOVL5 | 0,886 | 0,44 | 1,15E-02 | 0,074 |
| cg10524687 | 7 | 51148784 | COBL | 0,825 | 0,44 | 1,16E-02 | 0,093 |
| cg23448978 | 7 | 51209365 | COBL | 0,815 | 0,48 | 4,56E-03 | -0,169 |
| cg07880636 | 7 | 51384621 | COBL | 0,812 | 0,44 | 1,72E-02 | 0,021 |
| cg14740417 | 8 | 139600915 | COL22A1 | 0,919 | 0,44 | 1,49E-04 | 0,148 |
| cg26477221 | 10 | 13702163 | FRMD4A | 0,828 | 0,44 | 1,55E-02 | -0,085 |
| cg05104995 | 10 | 49460249 | FRMPD2 | 0,805 | 0,44 | 1,20E-02 | -0,136 |
| cg22670503 | 10 | 49482695 | FRMPD2 | 0,847 | 0,44 | 1,42E-03 | -0,161 |
| cg16396933 | 10 | 104954103 | NT5C2 | 0,825 | 0,44 | 8,93E-03 | -0,160 |
| cg15649702 | 11 | 34177094 | ABTB2 | 0,867 | 0,44 | 1,72E-03 | 0,139 |
| cg02697979 | 11 | 34265361 | ABTB2 | 0,873 | 0,44 | 2,90E-04 | -0,108 |
| cg23683201 | 11 | 63137152 | SLC22A9 | 0,828 | 0,52 | 2,37E-03 | 0,176 |
| cg12129012 | 11 | 64405346 | NRXN2 | 0,802 | 0,44 | 2,78E-03 | 0,131 |
| cg26805405 | 11 | 64491434 | NRXN2 | 0,847 | 0,44 | 5,17E-03 | 0,059 |
| cg26247168 | 11 | 119994722 | TRIM29 | 0,805 | 0,4 | 4,23E-03 | -0,162 |
| cg19056004 | 12 | 7023262 | LRRC23;ENO2 | 0,831 | 0,44 | 7,24E-03 | -0,118 |
| cg14210985 | 12 | 28115804 | PTHLH | 0,847 | 0,44 | 1,24E-02 | 0,048 |
| cg03626024 | 12 | 108524345 | WSCD2 | 0,808 | 0,48 | 6,30E-03 | 0,166 |
| cg17180088 | 12 | 108629501 | WSCD2 | 0,802 | 0,44 | 1,66E-02 | -0,029 |
| cg03799530 | 12 | 111843215 | SH2B3 | 0,815 | 0,44 | 3,36E-02 | -0,081 |
| cg00685314 | 12 | 120307689 | CIT | 0,812 | 0,44 | 3,67E-03 | -0,026 |
| cg03099988 | 12 | 132834467 | GALNT9 | 0,805 | 0,44 | 3,77E-03 | -0,082 |
| cg09258689 | 12 | 132853954 | GALNT 9; LOC100130238 | 0,857 | 0,44 | 5,62E-04 | 0,090 |
| cg18817318 | 13 | 77565875 | CLN5 | 0,851 | 0,44 | 1,97E-03 | -0,142 |
| cg19965589 | 15 | 52043121 | TMOD2;LYSMD2 | 0,834 | 0,44 | 9,20E-03 | 0,127 |
| cg27648738 | 15 | 84115811 | SH3GL3 | 0,825 | 0,44 | 2,83E-02 | -0,012 |
| cg08497530 | 16 | 82660434 | CDH13 | 0,864 | 0,44 | 2,66E-02 | 0,083 |
| cg01396387 | 16 | 82660450 | CDH13 | 0,828 | 0,44 | 4,27E-03 | 0,118 |
| cg01301138 | 16 | 82660630 | CDH13 | 0,815 | 0,44 | 2,95E-02 | 0,052 |
| cg08521677 | 17 | 8054688 | PERI | 0,851 | 0,48 | 1,59E-03 | -0,160 |
| cg16545079 | 17 | 8055888 | PERI | 0,841 | 0,44 | 2,97E-03 | -0,074 |
| cg02132714 | 17 | 46656690 | HOXB4 | 0,815 | 0,44 | 7,69E-03 | 0,140 |
| cg04293307 | 17 | 63553581 | AXIN2 | 0,831 | 0,4 | 2,51E-03 | -0,167 |
| cg19965023 | 17 | 72838366 | GRIN2C | 0,818 | 0,44 | 2,35E-03 | -0,111 |
| cg07015511 | 17 | 76497868 | DNAH17 | 0,841 | 0,44 | 5,90E-03 | -0,012 |
| cg24738140 | 17 | 76498535 | DNAH17 | 0,818 | 0,44 | 9,86E-03 | 0,150 |
| cg05845879 | 17 | 76507938 | DNAH17 | 0,805 | 0,44 | 7,68E-03 | -0,013 |
| cg13573245 | 19 | 5913990 | CAPS | 0,821 | 0,44 | 3,42E-03 | 0,145 |
| cg07886195 | 19 | 11263615 | SPC24 | 0,821 | 0,32 | 3,58E-04 | -0,212 |
| cg04753936 | 19 | 55141618 | LILRB1 | 0,870 | 0,44 | 1,29E-02 | -0,124 |
| cg02348449 | 19 | 58630429 | ZSCAN18 | 0,831 | 0,44 | 1,92E-02 | -0,116 |
| cg19155932 | 20 | 56725873 | C20orf85 | 0,805 | 0,44 | 5,40E-03 | 0,081 |
| cg00254133 | 20 | 61340542 | NTSR1 | 0,821 | 0,4 | 9,30E-03 | 0,151 |
| cg25037461 | 22 | 24181268 | DERL3 | 0,802 | 0,44 | 1,40E-02 | -0,078 |

| | | | | | | | |
|---|---|---|---|---|---|---|---|
| "PDCHA" stands for PDCHA complex (protocadherin alpha and subfamily C) and contains members PCDHA9, PCDHA6, PCDHA4, PCDHA13, PCDHAC1, PCDHA10, PCDHA8, PCDHA3, PCDHA1, PCDHA5, PCDHA12, PCDHAC2, PCDHA2, PCDHA7, PCDHA11. | | | | | | | |

### Figures:

Fig. 1: methylation profiles of selected markers distinguish benign vs. malignant (A) and FTA vs. FTC (B)). **Probe ids of tables 1 (Fig. A) and 2 (Fig. B) are given at the right side.**
Fig. 2: shows that one can draw as little as 6 randomly selected markers from the 126 CpG list (table 1) and still yield a median classification error rate below 15% for the distinction of malignant from benign thyroid nodules, which is the lowest error rate the best single genes have (PDZK1IP1, SORBS2). This rate drops to < 10% when increasing the marker number to > 20.
Fig. 3: shows that one can draw as little as 6 randomly selected markers from the 73 CpG list (table 2) and still yield a median classification error rate below 20% for the distinction of FTC from FTA, which is the lowest error rate the best single gene has (C1ORF21). This rate drops to < 10% when increasing the marker number to > 26 and 4% for using all markers.
Fig. 4: shows expression data of the genes of table 1 and provides expression levels for Struma nodosa (SN) FTA, FTC and PTC.
Fig. 5: shows expression of the genes of table 2 and provides expression levels for FTA and FTC

### Examples:

### Example 1: Material and Methods

### Patients and Samples

Fresh frozen thyroid nodules from 46 patients (10 PTC, 14 FTA, 11 FTC, 11 SN) were collected at the Medical University of Vienna, Department of Clinical Pathology in the years 1993-2009. Average age at surgery was 52±19 years. After surgery the thyroid tissue was immediately submerged in liquid nitrogen to preserve nucleic acid. The tissue samples were made anonymous and forwarded to AIT. This study was approved by the Ethics Committee of the Medical University of Vienna.

Sample quality and sample allocation was evaluated by a qualified pathologist. All samples provided sufficient amounts of high quality DNA (purity [260/280]: 1.7-2.2) for all downstream analysis.

### Tissue Processing and Analysis

A section of each sample was histologically examined by a pathologist to confirm the tumor entity and quality. Approximately 100 mg of tissue was used for DNA and mRNA isolation. Genomic DNA was isolated using the AllPrep DNA/RNA Mini-Kit (Qi-agen, Hilden, Germany) according to the manufacturer's protocol. DNA quantification was done on a Nanodrop 1000 upon absorbance measurements (260/280 nm).

### Genome-wide methylation assay

For whole genome methylation analysis, the Infinium 450k methylation platform (Illumina, USA) was used (Quantitative cross-validation and content analysis of the 450k DNA methylation array from Illumina, Inc. BioMed Central Ltd 2012.). Briefly, a total of 500 ng of genomic DNA was subjected to sodium bisulfite conversion using the EZ DNA Methylation Kit (Zymo Research, California, USA), following the manufacturers protocol with a slight adaption during the incubation protocol according to Illumina's recommendations. Instead of an isothermal incubation at 50 °C for 16h, a cycling incubation was used (16 cycles; 95 °C for 30 sec; 50 °C for 60 min; storage at 4 °C). The DNA was eluted in 12 µl elution buffer.

An aliquot of the converted DNA (4 µl) of the 48 samples was assayed by Illumina's HumanMethylation450k BeadChip, following the manufacturer's protocol. The remaining 8 µl were stored at - 20 °C as backup.

### Genome-wide gene expression assay

Briefly, 200 ng of total RNA was reverse transcribed. Amplification and labeling were performed by T7-polymerase in vitro transcription, to give Cy3-labeled cRNA. The dye incorporation rate was assessed with a Nanodrop ND-1000 spectrophotometer and was consistently >9 pmolCy3/ugRNA. Single color hybridization were carried out using the Agilent Gene Expression Hybridisation Kit (p/n 5188-5242), following the manufacturer's instructions. Briefly, 1650 ng of cRNA was subjected to fragmentation (30 min at 60C) and then hybridization on 4x44K Human Whole-Genome 60-mer oligo-chips (G4112F, Agilent Technologies) in a rotary oven (10 rpm, 65C, 17 h). Slides were disassembled and washed in solutions I and II according to the manufacturer's instructions, and dried using Acetonitril. Scanning was done on an Agilent microarray scanner (p/n G2565BA) followed by Agilent Feature Extraction Software.

### Data Extraction and data analysis

Results from the BeadChips were initially extracted by Illumina's BeadStudio software with the Methylation Module. Beta scores as well as detection p-values were generated in BeadStudio.

Data of both platforms (Methylation and Gene Expression) were analyzed within the R environment. Missing values were imputed using KNN-Impute (Class Prediction by Nearest Shrunken Centroids, with Applications to DNA Microarrays: The Institute of Mathematical Statistics; 2003). The data was quantile normalized before statistical evaluation.

For both methylation and gene expression data, differential methylation/expression analysis was performed using ANOVA models with empirical bayes moderated variances as implemented in the limma package (Bioconductor) (*Bioconductor: open software development for computational biology and bioinformatics:* BioMed Central). Similarly, ROC analysis was performed to assess the diagnostic relevance of the findings.

For the selection of relevant marker genes and CpG sites from the methylation data, an AUC-value (from ROC analysis) > 0.8 and an absolute beta-difference > 0.1 and a p-value < 0.05 (Benjamini Hochberg corrected) in methylation analysis and a p-value < 0.05 in expression analysis was chosen.

Selected markers were used to train classification models using a nearest centroid algorithm implemented in the PAMR package. In order to assess whether classification accuracies depend on the size of the gene panel used in classification, a random set of n genes from the pool of genes surviving the thresholds (AUC >0.8 AND absolute beta-difference > 0.1 AND p-value < 0.05 AND p-value in gene expression < 0.05, see above) was drawn and classification accuracies were determined in leave-one-out-cross-validation (loocv). This procedure was repeated 1000 times for each n.

### Example 2: Genome wide methylation analysis validation of the microarray data

The sample set was subjected to genome wide methylation analysis using the HumanMethylation450 BeadChip from Illumina. We selected genes according to the rules specified in in example 1 with the aim of selecting marker genes and CpG sites with strong differential methylation (beta difference, i.e. the difference between the methylation specific probe and methylation non-specific probe, and p-value), predictive power (AUC) and an effect on gene expression (p-value from gene expression).

This yielded the inventive marker sets, which contains markers with two specialties: markers which can distinguish between benign and malignant thyroid nodules and markers which distinguishes between FTA and FTC. The first subset of markers consists of 126 CpG sites which map to 63 genes (many genes represented by many CpG sites). The second subset of markers consists of 73 CpG sites which map to 65 genes. The tables 1 and 2 of methylated genes plus their graphical representation as boxplot and ROC curves are given above in the detailed description and illustrated in the figures. 11 genes are shared between these two tables, the rest is unique (ACOT7, C1orf21, PCNXL2, KCNAB1, ABLIM3, TREM1, COBL, WSCD2, CIT, AXIN2, SPC24).

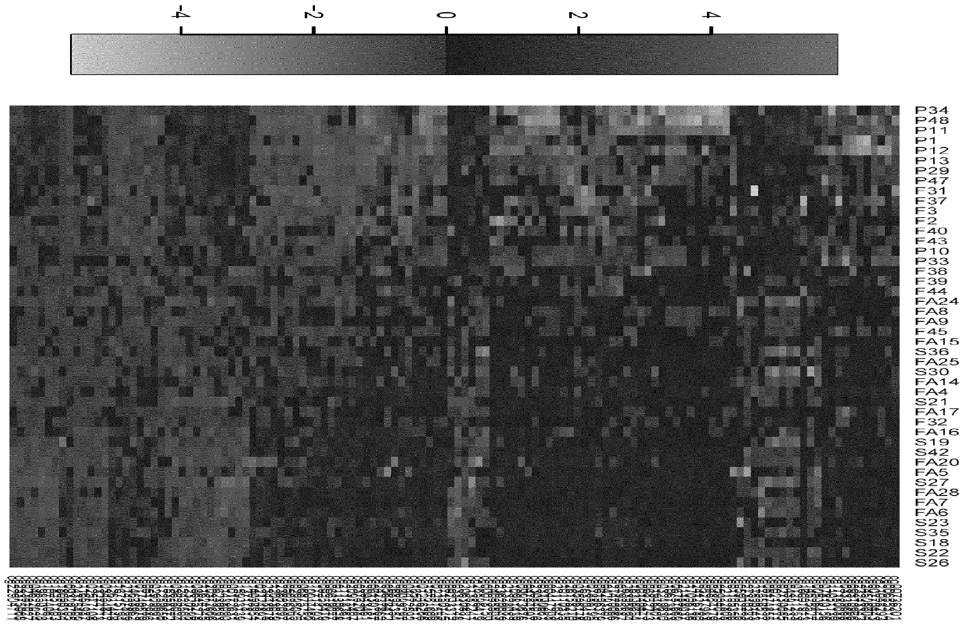

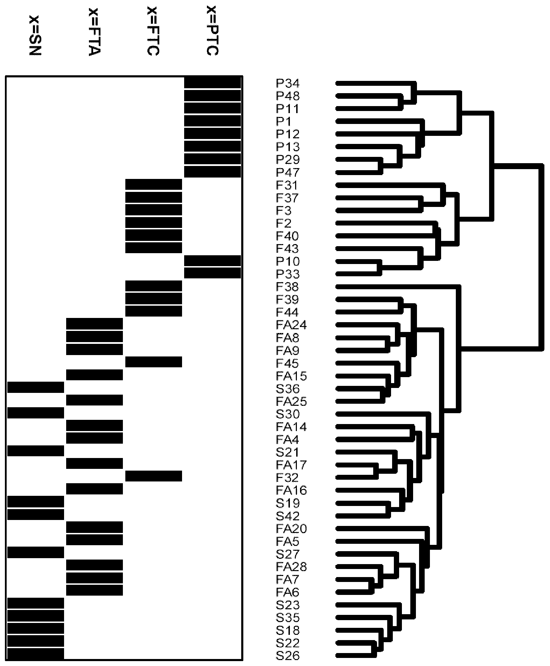

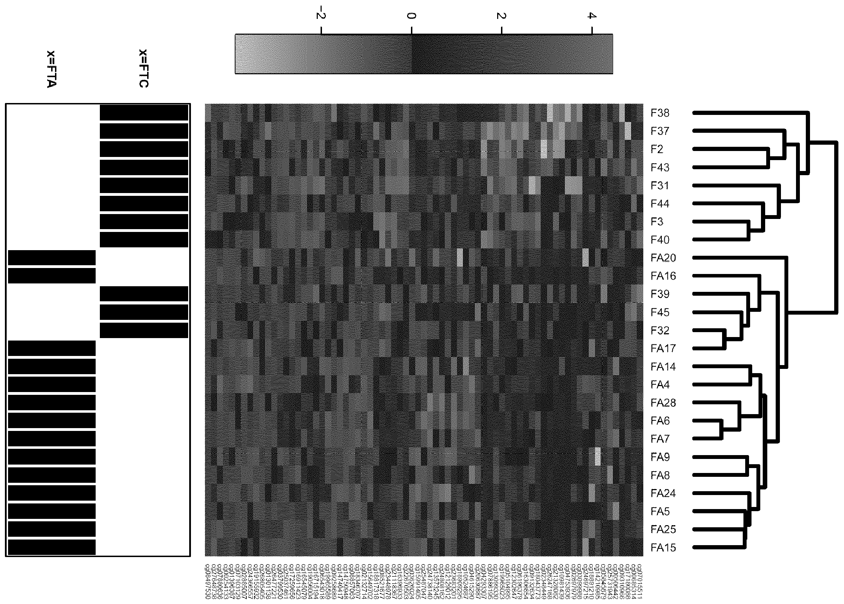

Unsupervised clustering based on these genes shows clear patterns of methylation which correlates to the histological endpoint used for analysis (Figure 1). Both approaches reveal a clear benign and a clear malignant cluster, but also shows a third, 'suspicious' cluster which is molecularly more similar to the benign group but contains samples which were classified histologically as malignant. In the case of the first set of features (benign vs malignant), this group consists of 0/10 PTC samples, 4/11 FTC, 5/14 FTA and 1/11 SN (struma nodosa, a benign thyroid nodule) samples. This reflects the current clinical situation, where the majority of misclassification by cytology are between FTA and FTC and raises important questions about the real malignancy of some of the FTC cases. Similarly, the second set of features (FTA vs FTC) shows a group of five samples with a molecular profile similar to the benign samples, but consisting of 3/11 FTC samples and 2/14 FTA samples.

### Example 3: Construction of gene sets with optimal classification accuracies

Owing to the complex nature of tumours on the one hand, and the redundancy in biological processes on the other hand,using only one gene or CpG site has a high risk. Therefore, two sets of markers in tables 1 and 2 (with 126 and 73 CpG sites, respectively) are provided, which greatly improve on single marker diagnosis. When a minimum of markers is drawn, a good classification accuracy is achieved - see Figs. 2 and 3. In order to find out how many of those markers allow optimal classification, a random selection of each number of markers was drawn and a leave-one-out-cross validation error rate was calculated using support vector machine classification. This procedure was repeated 1000 times for each gene panel size. The results are shown in Figure 2 and Figure 3.

For the classification task benign vs malign, 6 genes out of a total of 126 need to be drawn to yield a median misclassification rate of < 15%, which is the minimum of what the best single genes out of the pool can achieve (PDZK1IP1 or SORBS2). Similarly, for the task of predicting FTA vs FTC, also 6 genes need to be drawn out of the pool of 73 genes to yield a misclassification rate < 20%, which is the minimum of what the best single gene out of the pool can achieve (C1ORF21). Some markers of the inventive sets are also suitable for single marker diagnosis, but even in these cases, an improvement can be achieved by selecting more than one marker.

The drop in classification accuracy shown here is in stark contrast to recent work done by Rodriguez-Romero et. al. (J. Clin. Endocrinol. Metab. 2013, 98:2811-2821). They measured DNA methylation in thyroid nodules using the predecessor platform from Illumina which contained probes for 27000 CpG sites. They report 8613 CpG sites as differentially methylated at a p-value < 0.05, but do not report any diagnostically relevant values (accuracies, AUC-values, etc...). Furthermore, they do not report any combination of markers to be diagnostically relevant.

The result of the study is a novel set of biomarkers combined in two classifiers for correct prediction of benign and malignant thyroid nodules as well as for the discrimination of FTCs and FTAs. The set of biomarkers suggests that there are detectable epigenetic alterations which allow the identification of the different thyroid nodules entities. In contrast to other studies we did not focus exclusively on the 5'UTR region of the certain genes, but included any gene region for which an informative character was suggested by the microarray experiments and we included gene expression data to assess whether any methylation change has an effect on gene expression or not.

This allows the use of the biomarkers in the clinical routine setting. Furthermore the presented set of biomarkers based on DNA methylation is easier to handle and more amenable compared to biomarkers based on mRNA. Replacing or aiding cytology by an assay covering the newly defined set of biomarkers should result in fewer patients with indeterminate cases of thyroid nodules. That would also facilitate patients care by reducing unnecessary surgeries of indeterminate cases and increase patients care towards personalized medicine.

## Claims

1. A method of distinguishing a thyroid cancer type or risk thereof,
comprising the step of determining the DNA methylation status of at least 3 thyroid cancer genes of a sample of a subject, wherein the at least 3 thyroid cancer genes are selected from three or more of the genes of the proviso given below and/or of table 1 and/or table 2,
and comparing the methylation status of said genes with a control sample, thereby identifying thyroid cancer DNA in the sample,
with the proviso that at least one thyroid cancer gene is selected from TREM1, LRP2, NEK11, ABTB2, ACOT7, ADM, ALOX5, ANKRD22, AXIN2, BHLHE40, C10orf107, Clorf21, C20orf85, CAPS, CHKA, CIITA, CIT, CLN5, COBL, COL22A1, CPLX2, DERL3, DNAH17, DNAH9, ELM01, ELOVL5, ENO2, FAM20A, FMOD, FRMPD2, GALNT9, GJB6, GRIN2C, HK1, HLA-DOA, HOXD9, IFT140, IL17RD, IP6K3, ITM2C, ITPR1, KCNAB1, KCNN4, KRT80, LILRB1, LIPH, LOC100130238, LRRC23, LYSMD2, MACC1, MICALCL, MINA, MPPED2, MTSS1, MYO1G, NRXN2, NT5C2, NTSR1, PAG1, a PCDHA other than PCDHA13, PCNXL2, PCNXL2, PDZK1IP1, PDZRN4, PER1, PIM3, PRDM11, PRR7, PTHLH, PTPRF, RUNX2, SH2B3, SH3GL3, SLC22A9, SORBS2, SPC24, SUPT3H, SYN2, TFAP2B, TIMP4, TMC6, TMC8, TMEM204, TMOD2, TRIM29, UHRF1, WSCD2, ZSCAN18.

2. The method of claim 1 wherein the genes are selected from ABLIM3, ACOT7, ADM, ALOX5, ANKRD22, AXIN2, BHLHE40, C10orf107, Clorf21, CHKA, CIITA, CIT, COBL, CYB561, DNAH9, ELM01, EPHA10, FAM20A, FMOD, GJB6, HK1, IFT140, TMEM204, IL17RD, IP6K3, IRF5, ITPR1, KCNAB1, KCNN4, KLK10, KRT80, LIPH, LRP2, MACC1, MICALCL, MINA, MIOX, MPPED2, MTSS1, MYO1G, NEK11, PAG1, PCNXL2, PDZK1IP1, PDZRN4, PIM3, PRDM11, PRR7, RUNX2, SORBS2, SPC24, STRA6, SUPT3H, RUNX2, SYN2, TIMP4, TBX2, TMC6, TMC8, TREM1, UHRF1, WSCD2 and the distinguishing a thyroid cancer type or risk thereof is distinguishing a benign vs. a malignant state or the risk of a malignant state, wherein preferably the benign state comprises conditions FTA and normal and/or preferably the malignant state comprises conditions FTC and PTC.

3. A method of distinguishing FTA vs. FTC in a sample being suspected of having either FTA or FTC,
comprising the step of determining the DNA methylation status of at least 3 thyroid cancer genes of a sample of a subject,
wherein the at least 3 thyroid cancer genes are selected from three or more of the genes of table 2,
and comparing the methylation status of said genes with a FTA or FTC control sample.

4. The method of any one of claims 1 to 3, **characterized in that** the step of determining the methylation status comprises a methylation specific PCR analysis, methylation specific digestion analysis, preferably with hybridization analysis to non-digested or digested fragments, or PCR amplification analysis of non-digested or digested fragments, or bisulfite deamination followed by identification of methylated C changes, preferably by PCR or hybridization.

5. The method of any one of claims 1 to 4, **characterized in that** determining the methylation status comprises determining the methylation status of at least 4, preferably of 5, 6, 7, 8, 9, 10, 11, 12 or more of the genes of said table(s).

6. The method of any one of claims 1 to 5, comprising comparing the methylation status with the status of a confirmed thyroid cancer positive and/or negative state, preferably selected from a normal control, FTA, FTC and PTC, preferably wherein the control comprises a healthy thyroid nodule or no nodule.

7. The method of any one of claims 1 to 6, **characterized in that** determining the methylation status comprises determining the methylation status for at least 1, 2, 3, 4, 5, 6, 7, 8, 9, 10, 11, 12 or more genes in at least two potentially methylated regions of each gene.

8. The method of any one of claims 1 to 7, **characterized in that** determining the methylation status comprises comparing a methylation-status specific signal with a methylation-status unspecific signal at a preselected potentially methylated region of said gene.

9. The method of any one of claims 1 to 8, further comprising determining the gene expression of at least one of said genes of table 1 and/or 2, wherein a differential expression as compared to a normal sample indicates thyroid cancer or the risk thereof.

10. The method of any one of claims 1 to 9, **characterized in that** the methylation status of said genes is determined in an upstream region of the open reading frame of the marker genes, in particular a promoter region; or in a) a nucleic acid defined by the chromosomal locus as identified in table 1 or table 2; b) a CpG site encompassing the nucleic acid a); or c) a nucleic acid within at most 1000 nucleotides in length distanced from said nucleic acid a).

11. A kit comprising nucleic acid primers or hybridization probes being specific for a potentially methylated region of marker genes being suitable to diagnose or predict thyroid cancer, with the set comprising at least 3 probes and/or primers for genes: selected from three or more of the genes of table 1 and/or table 2, with the proviso that at least one thyroid cancer gene is selected from ABTB2, ACOT7, ADM, ALOX5, ANKRD22, AXIN2, BHLHE40, C10orf107, Clorf21, C20orf85, CAPS, CHKA, CIITA, CIT, CLN5, COBL, COL22A1, CPLX2, DERL3, DNAH17, DNAH9, ELM01, ELOVL5, ENO2, FAM20A, FMOD, FRMPD2, GALNT9, GJB6, GRIN2C, HK1, HLA-DOA, HOXD9, IFT140, IL17RD, IP6K3, ITM2C, ITPR1, KCNAB1, KCNN4, KRT80, LILRB1, LIPH, LOC100130238, LRP2, LRRC23, LYSMD2, MACC1, MICALCL, MINA, MPPED2, MTSS1, MYO1G, NEK11, NRXN2, NT5C2, NTSR1, PAG1, a PCDHA other than PCDHA13, PCNXL2, PCNXL2, PDZK1IP1, PDZRN4, PER1, PIM3, PRDM11, PRR7, PTHLH, PTPRF, RUNX2, SH2B3, SH3GL3, SLC22A9, SORBS2, SPC24, SUPT3H, SYN2, TFAP2B, TIMP4, TMC6, TMC8, TMEM204, TMOD2, TREM1, TRIM29, UHRF1, WSCD2, ZSCAN18, and the kit further comprises a computer readable memory device with a computer program product for performing the method of any one of claims 1 to 10, including a class comparison procedure to identify differentially methylated thyroid cancer genes from the set.

12. Kit according to claim 11, **characterized in that** the primer pairs and probes are specific for a methylated upstream region of the open reading frame of the marker genes, in particular a promoter region; or methylation in a) a nucleic acid defined by the chromosomal locus as identified in table 1 or table 2; b) a CpG site encompassing the nucleic acid a); or c) a nucleic acid within at most 1000 nucleotides in length distanced from said nucleic acid a).

13. Kit according to claim 11 or 12, **characterized in that** the set further comprises probes or primer specific for the potentially specific for a potentially methylated region of marker genes, wherein said further probes or primers are non-specific for DNA methylation and are suitable for use as a control or normalization agent.

14. Kit according to any one of claims 11 to 13, **characterized in that** the set is provided in a kit together with a methylation specific restriction enzyme and/or a reagent for bisulfite nucleotide deamination; and/or wherein the set comprises probes on a microarray.

15. Kit according to any one of claims 11 to 14, **characterized in that** the set contains at most 5000 probes or primers.

## Patentansprüche

1. Verfahren zur Unterscheidung eines Schilddrüsenkrebstyps oder Risikos dafür,
umfassend den Schritt des Bestimmens des DNA-Methylierungsstatus von mindestens 3 Schilddrüsenkrebsgenen aus einer Probe von einem Individuum,
wobei die mindestens 3 Schilddrüsenkrebsgene ausgewählt sind aus drei oder mehreren der Gene aus der unten angegebenen Maßgabe und/oder aus Tabelle 1 und/oder Tabelle 2,
und Vergleichen des Methylierungsstatus dieser Gene mit einer Kontrollprobe, dadurch Identifizieren von Schilddrüsenkrebs-DNA in der Probe,
mit der Maßgabe dass mindestens ein Schilddrüsenkrebsgen ausgewählt ist aus TREM1, LPR2, NEK11, ABTB2, ACOT7, ADM, ALOX5, ANKRD22, AXIN2, BHLHE40, C10orf107, C1orf21, C20orf85, CAPS, CHKA, CIITA, CIT, CLN5, COBL, COL22A1, CPLX2, DERL3, DNAH17, DNAH9, ELM01, ELOVL5, ENO2, FAM20A, FMOD, FRMPD2, GALNT9, GJB6, GRIN2C, HK1, HLA-DOA, HOXD9, IFT140, IL17RD, IP6K3, ITM2C, ITPR1, KCNAB1, KCNN4, KRT80, LILRB1, LIPH, LOC100130238, LRRC23, LYSMD2, MACC1, MICALCL, MINA, MPPED2, MTSS1, MYO1G, NRXN2, NT5C2, NTSR1, PAG1, ein anderes PCDHA als PCDHA13, PCNXL2, PCNXL2, PDZK1IP1, PDZRN4, PER1, PIM3, PRDM11, PRR7, PTHLH, PTPRF, RUNX2, SH2B3, SH3GL3, SLC22A9, SORBS2, SPC24, SUPT3H, SYN2, TFAP2B, TIMP4, TMC6, TMC8, TMEM204, TMOD2, TRIM29, UHRF1, WSCD2, ZSCAN18.

2. Verfahren nach Anspruch 1, wobei die Gene ausgewählt sind aus ABLIM3, ACOT7, ADM, ALOX5, ANKRD22, AXIN2, BHLHE40, C10orf107, Clorf21, CHKA, CIITA, CIT, COBL, CYB561, DNAH9, ELM01, EPHA10, FAM20A, FMOD, GJB6, HK1, IFT140, TMEM204, IL17RD, IP6K3, IRF5, ITPR1, KCNAB1, KCNN4, KLK10, KRT80, LIPH, LRP2, MACC1, MICALCL, MINA, MIOX, MPPED2, MTSS1, MYO1G, NEK11, PAG1, PCNXL2, PDZK1IP1, PDZRN4, PIM3, PRDM11, PRR7, RUNX2, SORBS2, SPC24, STRA6, SUPT3H, RUNX2, SYN2, TIMP4, TBX2, TMC6, TMC8, TREM1, UHRF1, WSCD2 und das Unterscheiden eines Schilddrüsenkrebstyps oder Risikos dafür ist das Unterscheiden eines benignen von einem malignen Status oder dem Risiko eines malignen Status, wobei vorzugsweise der benigne Status die Zustände FTA und normal umfasst und/oder vorzugsweise der maligne Status die Zustände FTC und PTC umfasst.

3. Verfahren zur Unterscheidung von FTA vs. FTC in einer Probe, die im Verdacht steht, entweder ein FTA oder FTC aufzuweisen,
umfassend den Schritt des Bestimmens des DNA-Methylierungsstatus von mindestens 3 Schilddrüsenkrebsgenen aus einer Probe von einem Individuum,
wobei die mindestens 3 Schilddrüsenkrebsgene ausgewählt sind aus drei oder mehreren der Gene aus Tabelle 2,
und Vergleichen des Methylierungsstatus dieser Gene mit einer FTA- oder FTC-Kontrollprobe.

4. Verfahren nach einem der Ansprüche 1 bis 3, **dadurch gekennzeichnet, dass** der Schritt des Bestimmens des Methylierungsstatus eine methylierungsspezifische PCR-Analyse, methylierungsspezifische Verdauanalyse, vorzugsweise mit Hybridisationsanalyse an nicht-verdaute oder verdaute Fragmente, oder PCR-Amplifikationsanalyse von nicht-verdauten oder verdauten Fragmenten, oder Bisulfit-Desaminierung, gefolgt von Identifizierung der Veränderungen von methyliertem C, vorzugsweise durch PCR oder Hybridisation, umfasst.

5. Verfahren nach einem der Ansprüche 1 bis 4, **dadurch gekennzeichnet, dass** das Bestimmen des Methylierungsstatus das Bestimmen des Methylierungsstatus von mindestens 4, vorzugsweise von 5, 6, 7, 8, 9, 10, 11, 12 oder mehr der Gene aus dieser/n Tabelle(n) umfasst.

6. Verfahren nach einem der Ansprüche 1 bis 5, umfassend das Vergleichen des Methylierungsstatus mit dem Status eines bestätigten Schilddrüsenkrebs-positiven und/oder -negativen Status, vorzugsweise ausgewählt aus einer normalen Kontrolle, FTA, FTC und PTC, vorzugsweise wobei die Kontrolle einen gesunden Schilddrüsenknoten oder keinen Knoten umfasst.

7. Verfahren nach einem der Ansprüche 1 bis 6, **dadurch gekennzeichnet, dass** das Bestimmen des Methylierungsstatus das Bestimmen des Methylierungsstatus für mindestens 1, 2, 3, 4, 5, 6, 7, 8, 9, 10, 11, 12 oder mehr Gene in mindestens zwei potenziell methylierten Regionen jedes Gens umfasst.

8. Verfahren nach einem der Ansprüche 1 bis 7, **dadurch gekennzeichnet, dass** das Bestimmen des Methylierungsstatus das Vergleichen eines Methylierungsstatusspezifischen Signals mit einem Methylierungsstatus-unspezifischen Signal an einer zuvor ausgewählten potenziell methylierten Region dieses Gens umfasst.

9. Verfahren nach einem der Ansprüche 1 bis 8, weiterhin umfassend das Bestimmen der Genexpression mindestens eines dieser Gene aus Tabelle 1 und/oder 2, wobei eine differenzielle Expression im Vergleich zu einer normalen Probe Schilddrüsenkrebs oder das Risiko dafür anzeigt.

10. Verfahren nach einem der Ansprüche 1 bis 9, **dadurch gekennzeichnet, dass** der Methylierungsstatus dieser Gene in einer Stromaufwärts-Region des offenen Leserahmens der Markergene bestimmt wird, insbesondere einer Promotorregion; oder in a) einer Nukleinsäure, definiert durch den chromosomalen Locus, wie in Tabelle 1 oder Tabelle 2 identifiziert; b) einer CpG-Stelle, welche die Nukleinsäure a) umfasst; oder c) einer Nukleinsäure im Abstand von innerhalb höchstens 1000 Nukleotiden Länge von der Nukleinsäure a).

11. Kit, umfassend Nukleinsäure-Primer oder Hybridisationssonden, die für eine potenziell methylierte Region von Markergenen spezifisch sind, wobei diese zur Diagnose oder Vorhersage von Schilddrüsenkrebs geeignet sind, wobei das Set mindestens 3 Sonden und/oder Primer für Gene umfasst, ausgewählt aus drei oder mehreren der Gene aus Tabelle 1 und/oder Tabelle 2, mit der Maßgabe dass mindestens ein Schilddrüsenkrebsgen ausgewählt ist aus ABTB2, ACOT7, ADM, ALOX5, ANKRD22, AXIN2, BHLHE40, C10orf107, C1orf21, C20orf85, CAPS, CHKA, CIITA, CIT, CLN5, COBL, COL22A1, CPLX2, DERL3, DNAH17, DNAH9, ELM01, ELOVL5, ENO2, FAM20A, FMOD, FRMPD2, GALNT9, GJB6, GRIN2C, HK1, HLA-DOA, HOXD9, IFT140, IL17RD, IP6K3, ITM2C, ITPR1, KCNAB1, KCNN4, KRT80, LILRB1, LIPH, LOC100130238, LRP2, LRRC23, LYSMD2, MACC1, MICALCL, MINA, MPPED2, MTSS1, MYO1G, NEK11, NRXN2, NT5C2, NTSR1, PAG1, einem anderen PCDHA als PCDHA13, PCNXL2, PCNXL2, PDZK1IP1, PDZRN4, PER1, PIM3, PRDM11, PRR7, PTHLH, PTRPF, RUNX2, SH2B3, SH3GL3, SLC22A9, SORBS2, SPC24, SUPT3H, SYN2, TFAP2B, TIMP4, TMC6, TMC8, TMEM204, TMOD2, TREM1, TRIM29, UHRF1, WSCD2, ZSCAN18,
und das Kit weiterhin ein computerlesbares Speichergerät mit einem Computerprogrammprodukt zur Durchführung des Verfahrens nach einem der Ansprüche 1 bis 10 umfasst, das ein Klassenvergleichsverfahren zur Identifizierung differenziell methylierter Schilddrüsenkrebsgene aus dem Set beinhaltet.

12. Kit gemäß Anspruch 11, **dadurch gekennzeichnet, dass** die Primerpaare und Sonden für eine methylierte Stromaufwärts-Region des offenen Leserahmens der Markergene spezifisch sind, insbesondere eine Promotorregion; oder Methylierung in a) einer Nukleinsäure, definiert durch den chromosomalen Locus, wie in Tabelle 1 oder Tabelle 2 identifiziert; b) einer CpG-Stelle, welche die Nukleinsäure a) umfasst; oder c) einer Nukleinsäure im Abstand von innerhalb höchstens 1000 Nukleotiden Länge von der Nukleinsäure a).

13. Kit gemäß Anspruch 11 oder 12, **dadurch gekennzeichnet, dass** das Set weiterhin Sonden oder Primer umfasst, die für eine potenziell methylierte Region der Markergene spezifisch sind, wobei diese weiteren Sonden oder Primer für DNA-Methylierung nichtspezifisch sind und zur Verwendung als ein Mittel zur Kontrolle oder Normalisierung geeignet sind.

14. Kit gemäß einem der Ansprüche 11 bis 13, **dadurch gekennzeichnet, dass** das Set in einem Kit zusammen mit einem methylierungsspezifischen Restriktionsenzym und/oder eine Reagenz für Bisulfit-Nukleotid-Desaminierung bereitgestellt wird; und/oder wobei das Set Sonden auf einem Mikroarray umfasst.

15. Kit gemäß einem der Ansprüche 11 bis 14, **dadurch gekennzeichnet, dass** das Set höchstens 5000 Sonden oder Primer enthält.

## Revendications

1. Procédé de différenciation d'un type de cancer de la thyroïde ou d'un risque de celui-ci,
comprenant l'étape consistant à déterminer l'état de méthylation de l'ADN d'au moins 3 gènes du cancer de la thyroïde d'un échantillon d'un sujet, où les au moins 3 gènes du cancer de la thyroïde sont sélectionnés parmi trois des gènes ou plus de la condition indiquée ci-dessous et/ou du tableau 1 et/ou tableau 2,
et à comparer l'état de méthylation desdits gènes avec un échantillon de contrôle, en identifiant ainsi l'ADN du cancer de la thyroïde dans l'échantillon,
à condition qu'au moins un gène du cancer de la thyroïde soit sélectionné parmi TREM1, LRP2, NEK11, ABTB2, ACOT7, ADM, ALOX5, ANKRD22, AXIN2, BHLHE40, C10orf107, C1orf21, C20orf85, CAPS, CHKA, CIITA, CIT, CLN5, COBL, COL22A1, CPLX2, DERL3, DNAH17, DNAH9, ELMO1, ELOVL5, ENO2, FAM20A, FMOD, FRMPD2, GALNT9, GJB6, GRIN2C, HK1, HLA-DOA, HOXD9, IFT140, IL17RD, IP6K3, ITM2C, ITPR1, KCNAB1, KCNN4, KRT80, LILRB1, LIPH, LOC100130238, LRRC23, LYSMD2, MACC1, MICALCL, MINA, MPPED2, MTSS1, MYO1G, NRXN2, NT5C2, NTSR1, PAG1, un PCDHA autre que PCDHA13, PCNXL2, PCNXL2, PDZK1IP1, PDZRN4, PER1, PIM3, PRDM11, PRR7, PTHLH, PTPRF, RUNX2, SH2B3, SH3GL3, SLC22A9, SORBS2, SPC24, SUPT3H, SYN2, TFAP2B, TIMP4, TMC6, TMC8, TMEM204, TMOD2, TRIM29, UHRF1, WSCD2, ZSCAN18.

2. Procédé selon la revendication 1, dans lequel les gènes sont sélectionnés parmi ABLIM3, ACOT7, ADM, ALOX5, ANKRD22, AXIN2, BHLHE40, C10orf107, C1orf21, CHKA, CIITA, CIT, COBL, CYB561, DNAH9, ELM01, EPHA10, FAM20A, FMOD, GJB6, HK1, IFT140, TMEM204, IL17RD, IP6K3, IRF5, ITPR1, KCNAB1, KCNN4, KLK10, KRT80, LIPH, LRP2, MACC1, MICALCL, MINA, MIOX, MPPED2, MTSS1, MYO1G, NEK11, PAG1, PCNXL2, PDZK1IP1, PDZRN4, PIM3, PRDM11, PRR7, RUNX2, SORBS2, SPC24, STRA6, SUPT3H, RUNX2, SYN2, TIMP4, TBX2, TMC6, TMC8, TREM1, UHRF1, WSCD2 et la différenciation d'un type de cancer de la thyroïde ou d'un risque de celui-ci est la différenciation entre un état bénin vs. malin ou le risque d'un état malin, où de préférence l'état bénin comprend des conditions de FTA et normales et/ou de préférence l'état malin comprend des conditions de FTC et PTC.

3. Procédé de différenciation d'un FTA vs. FTC dans un échantillon qui est suspecté de présenter un FTA ou un FTC,
comprenant l'étape consistant à déterminer l'état de méthylation de l'ADN d'au moins 3 gènes du cancer de la thyroïde d'un échantillon d'un sujet,
où les au moins 3 gènes du cancer de la thyroïde sont sélectionnés parmi trois des gènes ou plus du tableau 2,
et à comparer l'état de méthylation desdits gènes avec un échantillon de contrôle de FTA ou FTC.

4. Procédé selon l'une quelconque des revendications 1 à 3, **caractérisé en ce que** l'étape consistant à déterminer l'état de méthylation comprend une analyse de PCR spécifique de méthylation, une analyse de digestion spécifique de méthylation, de préférence avec une analyse d'hybridation à des fragments non digérés ou digérés, ou une analyse d'amplification par PCR de fragments non digérés ou digérés, ou une désamination par le bisulfite suivie d'une identification de modifications de C méthylées, de préférence par une PCR ou une hybridation.

5. Procédé selon l'une quelconque des revendications 1 à 4, **caractérisé en ce que** l'étape consistant à déterminer l'état de méthylation comprend la détermination de l'état de méthylation d'au moins 4, de préférence de 5, 6, 7, 8, 9, 10, 11, 12 des gènes ou plus dudit/desdits tableau(x).

6. Procédé selon l'une quelconque des revendications 1 à 5, comprenant la comparaison de l'état de méthylation avec l'état d'un état positif et/ou négatif pour un cancer de la thyroïde confirmé, de préférence sélectionné parmi un contrôle normal, un FTA, un FTC et un PTC, de préférence où le contrôle comprend un nodule thyroïdien sain ou aucun nodule.

7. Procédé selon l'une quelconque des revendications 1 à 6, **caractérisé en ce que** la détermination de l'état de méthylation comprend la détermination de l'état de méthylation pour au moins 1, 2, 3, 4, 5, 6, 7, 8, 9, 10, 11, 12 gènes ou plus dans au moins deux régions potentiellement méthylées de chaque gène.

8. Procédé selon l'une quelconque des revendications 1 à 7, **caractérisé en ce que** la détermination de l'état de méthylation comprend la comparaison d'un signal spécifique de l'état de méthylation avec un signal non spécifique de l'état de méthylation au niveau d'une région potentiellement méthylée présélectionnée dudit gène.

9. Procédé selon l'une quelconque des revendications 1 à 8, comprenant en outre la détermination de l'expression génique d'au moins un desdits gènes du tableau 1 et/ou 2, dans lequel une expression différentielle par comparaison à un échantillon normal indique un cancer de la thyroïde ou le risque de celui-ci.

10. Procédé selon l'une quelconque des revendications 1 à 9, **caractérisé en ce que** l'état de méthylation desdits gènes est déterminé dans une région en amont du cadre de lecture ouvert des gènes marqueurs, en particulier une région de promoteur ; ou dans a) un acide nucléique défini par le locus chromosomique tel qu'identifié dans le tableau 1 ou le tableau 2 ; b) un site CpG englobant l'acide nucléique a) ; ou c) un acide nucléique qui est éloigné d'une longueur de 1 000 nucléotides au maximum dudit acide nucléique a).

11. Kit comprenant des amorces d'acide nucléique ou des sondes d'hybridation qui sont spécifiques pour une région potentiellement méthylée de gènes marqueurs qui sont appropriés pour diagnostiquer ou prédire le cancer de la thyroïde, l'ensemble comprenant au moins 3 sondes et/ou amorces pour des gènes : sélectionnés parmi trois des gènes ou plus du tableau 1 et/ou tableau 2,
à condition qu'au moins un gène du cancer de la thyroïde soit sélectionné parmi ABTB2, ACOT7, ADM, ALOX5, ANKRD22, AXIN2, BHLHE40, C10orf107, C1orf21, C20orf85, CAPS, CHKA, CIITA, CIT, CLN5, COBL, COL22A1, CPLX2, DERL3, DNAH17, DNAH9, ELM01, ELOVL5, ENO2, FAM20A, FMOD, FRMPD2, GALNT9, GJB6, GRIN2C, HK1, HLA-DOA, HOXD9, IFT140, IL17RD, IP6K3, ITM2C, ITPR1, KCNAB1, KCNN4, KRT80, LILRB1, LIPH, LOC100130238, LRP2, LRRC23, LYSMD2, MACC1, MICALCL, MINA, MPPED2, MTSS1, MYO1G, NEK11, NRXN2, NT5C2, NTSR1, PAG1, un PCDHA autre que PCDHA13, PCNXL2, PCNXL2, PDZK1IP1, PDZRN4, PER1, PIM3, PRDM11, PRR7, PTHLH, PTPRF, RUNX2, SH2B3, SH3GL3, SLC22A9, SORBS2, SPC24, SUPT3H, SYN2, TFAP2B, TIMP4, TMC6, TMC8, TMEM204, TMOD2, TREM1, TRIM29, UHRF1, WSCD2, ZSCAN18,
et le kit comprend en outre un dispositif de mémoire lisible par un ordinateur avec un produit de programme informatique pour réaliser le procédé selon l'une quelconque des revendications 1 à 10, incluant une procédure de comparaison de classes pour identifier des gènes du cancer de la thyroïde différentiellement méthylés à partir de l'ensemble.

12. Kit selon la revendication 11, **caractérisé en ce que** les paires d'amorces et les sondes sont spécifiques pour une région en amont méthylée du cadre de lecture ouvert des gènes marqueurs, en particulier une région de promoteur ; ou une méthylation dans a) un acide nucléique défini par le locus chromosomique tel qu'identifié dans le tableau 1 ou le tableau 2 ; b) un site CpG englobant l'acide nucléique a) ; ou c) un acide nucléique qui est éloigné d'une longueur de 1 000 nucléotides au maximum dudit acide nucléique a).

13. Kit selon la revendication 11 ou 12, **caractérisé en ce que** l'ensemble comprend en outre des sondes ou des amorces spécifiques pour une région potentiellement méthylée des gènes marqueurs, dans lequel lesdites sondes ou amorces supplémentaires sont non spécifiques pour une méthylation de l'ADN et sont appropriées pour être utilisées en tant que contrôle ou agent de normalisation.

14. Kit selon l'une quelconque des revendications 11 à 13, **caractérisé en ce que** l'ensemble est fourni dans un kit avec une enzyme de restriction spécifique de méthylation et/ou un réactif pour une désamination de nucléotides par le bisulfite ; et/ou dans lequel l'ensemble comprend des sondes sur un microréseau.

15. Kit selon l'une quelconque des revendications 11 à 14, caractérisé en ce l'ensemble contient au maximum 5 000 sondes ou amorces.
